# EUROPEAN PATENT APPLICATION

(11) **EP 1 589 101 A1**
(43) Date of publication of application: **26.10.2005**
(21) Application number: 03768371.1
(22) Date of filing: 26.12.2003
(51) Int. Cl.: C12N 15/09, G01N 33/53

(54) **NOVEL PROTEIN AND ITS GENE**

(30) Priority: 27.12.2002 JP 2002382418
(71) Applicant: Chugai Pharmaceutical Co., Ltd., Tokyo 115-8543 (JP); TOUDAI TLO, Ltd., Tokyo 113-0033 (JP)
(72) Inventor: ABURATANI, Hiroyuki, Musashino-shi, Tokyo 180-0003 (JP); ITO, Hirotaka, Tokyo 157-0073 (JP); ISHIKAWA, Shunpei, Tokyo 153-0041 (JP); FUKUMOTO, Shinichi, Tokyo 153-0041 (JP)
(74) Representative: Bohnenberger, Johannes
(86) International application number: PCT/JP2003/017064
(87) International publication number: WO 2004/061103

(57) **Abstract**

With the object of providing a novel protein expression of which is specifically elevated in abnormal cells or abnormal tissue, and also providing a diagnostic method and diagnostic kit for diseases involving elevated expression of a gene encoding that protein, along with a screening method and screening kit for substances for preventing and treating diseases involving elevated expression of a gene encoding that protein, (a) a protein comprising the amino acid sequence represented by Seq. ID No. 2 or (b) a protein comprising the amino acid sequence represented by Seq. ID No. 2 with one or more amino acids deleted replaced or added is provided as a novel protein expression of which is specifically elevated in abnormal cells or abnormal tissue, with a protein expression of which is specifically elevated in abnormal cells or abnormal tissue, the level of expression of a gene encoding that protein is taken as a marker for diagnosing a disease and a reduction effect on the level of expression of a gene encoding that protein is taken as a marker for screening preventative and therapeutic substances for a disease.

## Description

### TECHNICAL FIELD

The present invention relates to a protein the expression of which is specifically elevated in abnormal cells or abnormal tissues (particularly lung cancer cells or lung cancer tissues), to a gene encoding the protein, to a recombinant vector comprising the gene, to a transformant comprising the recombinant vector, to an antibody or fragment thereof to the protein, to a diagnostic method and diagnostic kit in which the expression level of the gene is used as the indicator for diagnosing diseases (particularly lung cancer) involving elevated expression of the gene, and to a screening method and screening kit in which a reduction effect on the expression level of the gene is used as the indicator for screening substances for preventing and treating diseases (particularly lung cancer) involving elevated expression of the gene.

### BACKGROUND ART

Lung cancer is on the rise worldwide, and in 2015 there are expected to be 110,000 new cases among men and 37,000 among women in Japan. In 1999 the annual number of deaths from lung cancer in Japan was about 52,000, and since 1993 lung cancer has been the leading cause of cancer death among men and the second among women after stomach cancer. The 5-year survival rate (the rate of survival during the five years following commencement of therapy) for lung cancer is said to be 25 to 30%, so society has a need for effective therapeutic drugs for lung cancer.

There is also currently widespread demand for easy and sensitive methods of diagnosing lung cancer. In particular, one promising new diagnostic method is gene diagnosis, and attempts have been made to apply this to primary prevention by discovering groups with high risk factors, secondary prevention by early detection of lung cancer, detection of micrometastatic cells in peripheral blood, bone marrow or lymph node, and prognosis by evaluation of malignancy (Hisanobu Niitani, Lung Cancer Care Handbook 2^{nd} Ed., 2001).

Gene analysis techniques using DNA chips and the like have been developed in recent years, and comprehensive and inclusive cancer gene expression analysis is becoming practical. Gene groups involved in cancers becoming malignant due to multi-stage factors and in cancer cell invasion, metastasis and the like are being identified comprehensively by analyzing changes in expressed amounts of mRNA in cancer tissue using DNA chip analysis. Moreover, it is hoped that elucidation of the individual physiological functions of identified gene groups will provide numerous new findings about the properties of new cancer cells, and efforts are being made to identify molecules expression of which is elevated or reduced in various tumors. Lung cancer is also a target of gene expression analysis, and gene groups with elevated expression and gene groups with reduced expression have been identified (Bhattacharjee, A. et al, *Proceedings of the National Academy of Sciences of the United States of America* 2001, 98, p. 13790-13795; Nacht, M. et al, *Proceedings of the National Academy of Sciences of the United States of America* 2001, 98, p. 15203-15208; Chen, J.J.W. et al, *Cancer Research* 2001, 61, p. 5223-5230; Beer, D.G. et al, *Nature Medicine* 2002, 8, p. 816-824). As a result of comprehensive gene expression analysis, there have been reports of differences in expression pattern between different types of lung cancer, characteristic gene expression control in metastatic cancer and identification of gene groups useful for prognosis. However, molecules which are specifically expressed in lung cancer have not been discovered, and at present no lung cancer-specific target molecules or marker molecules useful for prognosis have been discovered with any promise of clinical effectiveness.

### DISCLOSURE OF THE INVENTION

It is an object of the present invention to provide first a protein the expression of which is specifically elevated in abnormal cells or abnormal tissue (particular lung cancer cells or lung cancer tissue), a gene encoding the protein, a recombinant vector comprising the gene, a transformant comprising the recombinant vector and an antibody or fragment thereof to the protein.

It is a second object of the present invention to provide a diagnostic method and diagnostic kit in which the expression level of a gene encoding a protein the expression of which is specifically elevated in abnormal cells and abnormal tissue (particularly lung cancer cells and lung cancer tissue) is used as the indicator for diagnosing diseases (particularly lung cancer) which involve elevated expression of the aforementioned gene.

Further, it is a third object of the present invention to provide a screening method and screening kit in which a reduction effect on the level of expression of a gene encoding a protein the expression of which is specifically elevated in abnormal cells and abnormal tissue (particular lung cancer cells and lung cancer tissue) is used as the indicator for screening substances for preventing and treating diseases (particularly lung cancer) which involve elevated expression of the aforementioned gene.

In order to achieve the aforementioned objects, the present invention provides the following protein, gene, recombinant vector, transformant, antibody or fragment thereof, diagnostic method and diagnostic kit and screening method and screening kit.
(1) A protein shown in (a) or (b) below.
   (a) A protein comprising the amino acid sequence represented by Seq. ID No. 2
   (b) A protein comprising the amino acid sequence represented by Seq. ID No. 2 with 1 or more amino acids deleted, replaced or added, the expression of which is specifically elevated in abnormal cells or abnormal tissue.
(2) The protein according to (1) above wherein the abnormal cells or abnormal tissue are lung cancer cells or lung cancer tissue.
(3) A gene encoding a protein according to (1) or (2) above.
(4) The gene according to (3) above comprising DNA shown in (c) or (d) below.
   (c) DNA comprising the sequence of nucleotides 103 through 1488 in the nucleotide sequence represented by Seq. ID NO. 1
   (d) DNA which hybridizes under stringent conditions with DNA complementary to the DNA shown in (c) above, and which encodes a protein the expression of which is specifically elevated in abnormal cells or abnormal tissue.
(5) A recombinant vector comprising the gene according to (3) or (4) above.
(6) A transformant comprising the recombinant vector according to (5) above.
(7) An antibody or fragment thereof capable of reacting to the protein according to (1) or (2) above.
(8) A diagnostic method for a disease involving elevated expression of a gene encoding the protein according to (1) above, comprising a step of using as the indicator the level of expression of the gene in a specimen collected from a test animal to determine whether or not the test animal suffers from the disease.
(9) The diagnostic method according to (8) above, comprising a step of measuring the level of expression based on the amount of mRNA encoding the protein according to (1) above which is present in the specimen.
(10) The diagnostic method according to (8) above, comprising a step of measuring the level of expression based on the amount of the protein according to (1) above which is present in the specimen.
(11) The diagnostic method according to any of (8) through (10) above, wherein the disease is lung cancer.
(12) A diagnostic kit for a disease involving elevated expression of a gene encoding the protein according to (1) above, comprising an oligonucleotide or polynucleotide capable of hybridizing with a nucleic acid encoding the protein according to (1) above.
(13) A diagnostic kit for a disease involving elevated expression of a gene encoding the protein according to (1) above, comprising an antibody or fragment thereof capable of reacting to the protein according to (1) above.
(14) The diagnostic kit according to (12) or (13) above, wherein the disease is lung cancer.
(15) A screening method for substances for preventing or treating a disease involving elevated expression of a gene encoding the protein according to (1) above, comprising a step of evaluating the preventative and therapeutic effects of candidate substances on the disease using as the indicator the reduction effect on the level of expression of the gene in cells or tissue in which the gene is highly expressed.
(16) The screening method according to (15) above, wherein the disease is lung cancer.
(17) A screening kit for substances for preventing or treating a disease involving elevated expression of a gene encoding the protein according to (1) above, comprising an oligonucleotide or polynucleotide capable of hybridizing with a nucleic acid encoding the protein.
(18) A screening kit for substances for preventing or treating a disease involving elevated expression of a gene encoding the protein according to (1) above, comprising an antibody or fragment capable of reacting to the protein.
(19) The screening kit according to (17) or (18) above, wherein the disease is lung cancer.

### BRIEF DESCRIPTION OF THE DRAWINGS

Figure 1 shows the expressed amounts of mRNA in human pulmonary adenocarcinoma and human normal lung tissue which react with a 230349_at_u133B probe.
Figure 2 shows the expressed amounts of mRNA in human normal tissues which react with a 230349_at_u133B probe.
Figure 3 shows the results of electrophoresis of a DNA fragment obtained by RACE (Rapid amplification cDNA ends).
Figure 4 is a chart of alignment results for LOC139320 and the nucleotide sequence of gene #15.
Figure 5 is a chart (continuation of Figure 4) of alignment results for LOC139320 and the nucleotide sequence of gene #15.
Figure 6 shows the presence and absence of gene #15 and LOC139320 expression in pulmonary adenocarcinoma tissue.
Figure 7 shows the presence and absence of gene #15 expression in pulmonary adenocarcinoma tissue (12 cases) and normal lung tissue (4 cases).
Figure 8 shows the presence and absence of gene #15 expression in cancer cells isolated by microdissection from pulmonary adenocarcinoma tissue.
Figure 9 shows the presence and absence of gene #15 expression in active or inactive monocytes or lymphocytes and human normal tissue.
Figure 10,shows the presence or absence of gene #15 expression in human stomach cancer, hepatoma and colon cancer.
Figure 11 shows alignment results for the amino acid sequence of a protein encoded by gene #15 and the amino acid sequence of the human XK protein.
Figure 12 shows alignment results for the amino acid sequence of a protein encoded by gene #15 and the amino acid sequence of the nematode Ced8 protein.

### BEST MODE FOR CARRYING OUT THE INVENTION

The present invention is explained in detail below.

The protein of the present invention is the protein shown in (a) and (b) below.
(a) A protein comprising the amino acid sequence represented by Seq. ID No. 2 (hereunder, "protein (a)").
(b) A protein comprising the amino acid sequence represented by Seq. ID No. 2 with one or more amino acids deleted, replaced and added, the expression of which is specifically elevated in abnormal cells or abnormal tissue (hereunder, "protein (b)").

Protein (a) or (b) is a protein the expression of which is specifically elevated in abnormal cells or abnormal tissue (including abnormal organs). "Specifically elevated in abnormal cells or abnormal tissue" signifies here that expression is not elevated in normal cells or normal tissues but only in abnormal cells or abnormal tissues. Moreover, "abnormal cells or abnormal tissues" signifies cells or tissues exhibiting some abnormal state in association with some disease, and there are no limits on the type as long as expression of protein (a) or (b) is elevated, but examples include lung cancer cells, lung cancer tissue and the like, and examples of lung cancer include pulmonary adenocarcinoma, pulmonary squamous cell carcinoma, large cell pulmonary carcinoma, small cell pulmonary carcinoma and the like. Since elevated expression of protein (a) or (b) is not observed in cells or tissues derived from cancers such as stomach cancer, hepatoma and colon cancer, expression of protein (a) or (b) is thought to be specifically elevated in lung cancer of the cancers in particular. Moreover, of the lung cancers expression of protein (a) or (b) is thought to be specifically elevated in pulmonary adenocarcinoma cells or pulmonary adenocarcinoma tissue in particular.

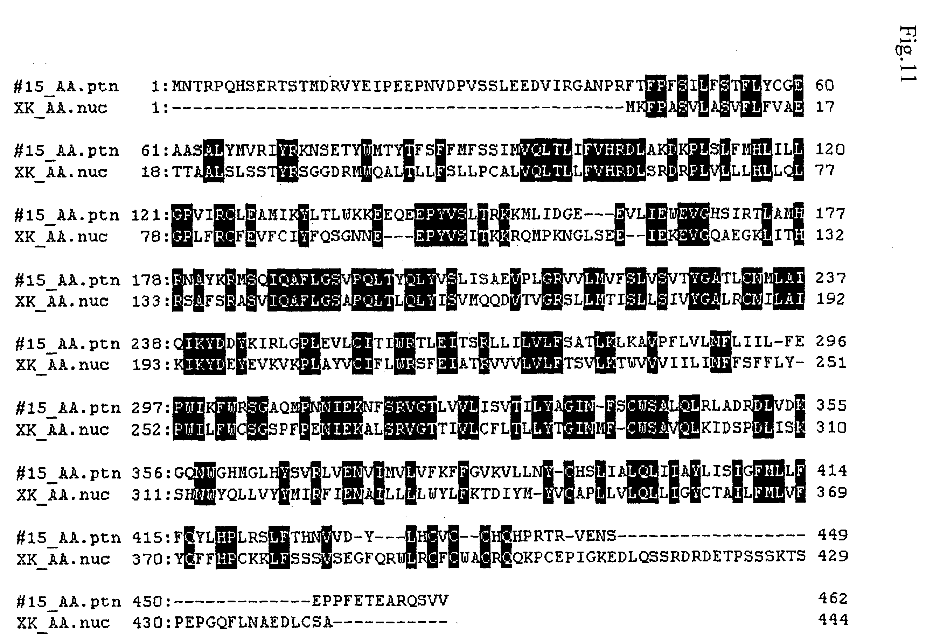

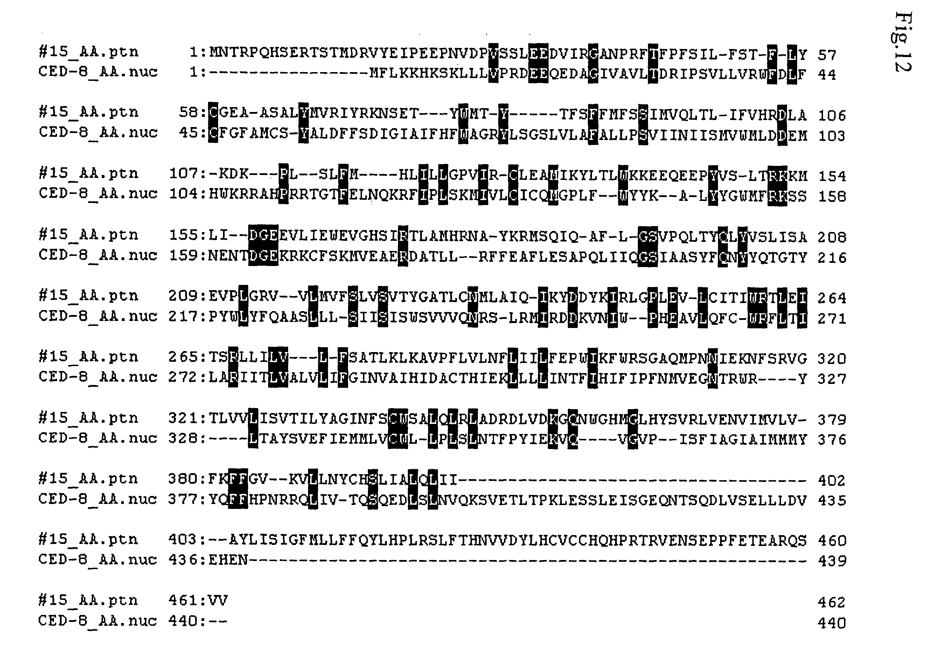

Protein (a) exhibits 44.7% and 19.4% homology, respectively, with the human XK protein (Kell blood group precursor gene) and the nematode CED8 protein at the amino acid level (see Figures 11 and 12). The human XK protein and nematode CED8 protein are both expressed in cell membranes and the like, and are predicted to function as transporters. The human XK protein is thought to have the function of transporting all or some Kell antigen protein precursors in red blood cells, and it is thought that human XK protein mutations cause XK protein dysfunction, so that Kell antigens disappear from the red blood cell surfaces, inducing morphological abnormalities of the red blood cells and ultimately causing acanthocytosis (Ho, M. et al, *Cell* Vol. 77, 869-880, 1994). Moreover, it is presumed from the fact that muscle disorders and neural disorders are seen in Mcleod's syndrome in addition to red blood cell abnormalities that the XK protein is also involved in the transport of neurotransmitters and the like (Danek, A. et al, *Ann. Neurol,* Vol. 28, 720-722, 1990; Danek, A. et al, *Ann. Neurol* Vol. 50, 755-764, 2001). Like the XK protein the nematode CED8 protein has a 10-transmembrane region and is thought to have a transporter structure, suggesting that it is localized on the cell membrane. Moreover, since the time for programmed cell death is delayed when the CED8 function is deficient at the initial stage of nematode lung development, it is thought to be an apoptosis control factor, and it has been suggested that the CED8 protein is involved in cell death by functioning either downstream from or at the same time as the CED9 protein, which functions in apoptosis regulation in nematodes (Stanfield, G.M. et al, *Molecular Cell* Vol. 5, 423-433, 2000). Anticipating the structure and function of the protein of the present invention based on the structures and functions of the XK protein and CED8, the protein of the present invention is predicted to function as a transporter with multiple transmembrane regions, and may be involved in apoptosis control like the CED8 protein, or may participate in the development and progress of pulmonary adenocarcinoma by transporting substances important for canceration of pulmonary adenocarcinoma or substances necessary for proliferation and survival of cancer cells.

There are no particular limits on the number of amino acids deleted, substituted or added in the amino acid sequence represented by Seq. ID No. 2 as long as expression is specifically elevated in abnormal cells or abnormal tissues (particularly lung cancer cells and lung cancer tissues), and the number is one or more or preferably one or a few, with the specific range being normally 1 to 100 or preferably 1 to 50 ore more preferably 1 to 10. In this case, the amino acid sequence of protein (b) normally has 15% or greater or preferably 40% or greater or more preferably 70% or greater homology with the amino acid sequence of protein (a).

There are no particular limits on the positions of amino acids deleted, substituted or added in the amino acid sequence represented by Seq. ID No. 2 as long as expression is specifically elevated in abnormal cells or abnormal tissues (particularly lung cancer cells and lung cancer tissues). For example, in the amino acid sequence represented by Seq. ID No. 2 the 9^{th} amino acid Glu can be replaced by the amino acid Gly, the 10^{th} amino acid Arg by the amino acid Gly, the 13^{th} amino acid Thr by the amino acid Ala, the 25^{th} amino acid Asn by the amino acid Asp, the 26^{th} amino acid Val by the amino acid Ala, the 29^{th} amino acid Val by the amino acid Asp, the 76^{th} amino acid Glu by the amino acid Gly, the 83^{rd} amino acid Thr by the amino acid Ala, the 90^{th} amino acid Ser by the amino acid Pro, the 111^{st} amino acid Leu by the amino acid Pro, the 112^{nd} amino acid Ser by the amino acid Pro, the 116^{th} amino acid His by the amino acid Arg, the 128^{th} amino acid Glu by the amino acid Lys, the 145^{th} amino acid Pro by the amino acid Ser, the 184^{th} amino acid Met by the amino acid Thr, the 200^{th} amino acid Gln by the amino acid Arg, the 227^{th} amino acid Tyr by the amino acid Cys, the 241^{st} amino acid Tyr by the amino acid Cys, the 259^{th} amino acid Trp by the amino acid Arg, the 296^{th} amino acid Glu by the amino acid Gly, the 308^{th} amino acid Met by the amino acid Thr, the 331^{st} amino acid Leu by the amino acid Ser, the 354^{th} amino acid Asp by the amino acid Gly, the 369^{th} amino acid Arg by the amino acid Lys, the 386^{th} amino acid Lys by the amino acid Glu, the 400^{th} amino acid Leu by the amino acid Phe, the 405^{th} amino acid Leu by the amino acid Pro, the 422^{nd} amino acid Arg by the amino acid Cys, and the 423^{rd} amino acid Ser by the amino acid Pro. Both proteins have replacements in one of the above replacement sites and proteins having replacements in any 2 or more are included in protein (b).

Protein (b) includes not only proteins having deletions, substitutions, additions and other mutations artificially introduced into protein (a), but also proteins naturally occurring with deletions, substitutions, additions and other introduced mutations or such proteins with deletions, substitutions, additions and other mutations artificially introduced. Examples of proteins naturally occurring with deletions, substitutions, additions and other introduced mutations include proteins (including proteins which may occur due to polymorphisms in such mammals) derived from mammals including humans (such as humans, monkeys, cows, sheep, goats, horses, pigs, rabbits, dogs, cats, mice, rats and the like).

Proteins (a) and (b) include proteins with added sugar chains and proteins without added sugar chains. The types, locations and the like of sugar chains added to the proteins will differ depending on the type of host cells used in manufacturing the protein, but proteins with added sugar chains include proteins obtained using any host cells. Moreover, proteins (a) and (b) include pharmacologically allowable salts thereof.

A gene encoding protein (a) or (b) is obtained for example by preparing a cDNA library using mRNA extracted from the lung cancer cells or lung cancer tissue of mammals including humans, and screening clones comprising the target DNA from the cDNA library using a probe synthesized based on the nucleotide sequence represented by Seq. ID No. 1. The steps of preparing the cDNA library and screening clones comprising the target DNA are explained below.

### (Preparation of cDNA library)

To prepare a cDNA library, for example total RNA is first obtained from the lung cancer cells or lung cancer tissue of mammals including humans, and poly(A+)RNA (mRNA) is then obtained by the batch method or affinity column method or the like using oligo dT-cellulose or poly U-sepharose. Poly(A+) RNA (mRNA) can also be fractioned by sucrose density gradient centrifugation or the like. Next, the resulting mRNA is used as the template for synthesizing single-strand cDNA using oligo dT primer and reverse transcriptase, after which double-strand cDNA is synthesized from the single-strand cDNA. A recombinant vector is prepared by incorporating the resulting double-strand cDNA into an appropriate cloning vector, *E. coli* or other host cells are transformed using the recombinant vector, and a cDNA library is obtained by selecting transformants using tetracycline resistance or ampicillin resistance as the marker. The cloning vector for preparing the cDNA library may be any capable of independent replication in host cells, and for example a phage vector, plasmid vector or the like can be used. *Escherichia coli* cells or the like for example can be used as the host cells.

Transformation of *E. coli* or other host cells can be accomplished for example by a method of adding the recombinant vector to competent cells prepared in the presence of calcium chloride, magnesium chloride or rubidium chloride. When a plasmid is used as the vector, it is desirable to include therein a tetracycline, ampicillin or other drug-resistance gene.

A commercial kit such as for example the SuperScript Plasmid System for cDNA Synthesis and Plasmid Cloning (Gibco BRL) or ZAP-cDNA Synthesis Kit (Stratagene) can be used in preparing the cDNA library.

### (Screening of clones comprising the target DNA)

To screen clones comprising the target DNA from the cDNA library, a primer is synthesized based on the nucleotide sequence represented by Seq. ID No. 1, and used in a polymerase chain reaction (PCR) to obtain PCR amplified fragments. The PCR amplified fragments can be sub-cloned using an appropriate plasmid vector. There are no particular limits on the primer set used in PCR, which can be designed based on the nucleotide sequence represented by Seq. ID No. 1.

The target DNA is obtained by colony hybridization or plaque hybridization of the cDNA library using the PCR amplified fragments as the probe. The PCR amplified fragments are labeled with an isotope (such as ³²P or ³⁵S), biotin, digoxigenin, alkaline phosphatase or the like for use as the probe. Clones comprising the target DNA can be obtained by expression screening such as immuno-screening using antibodies or the like.

Once the DNA fragments have been incorporated into a vector by normal means, either as is or after nicking with an appropriate restriction enzyme or the like, the nucleotide sequence of the obtained DNA can be determined by a commonly-used method of nucleotide sequence analysis such as the Maxam-Gilbert chemical modification method or the dideoxynucleotide chain termination method. A 373A DNA sequencer (Perkin Elmer) or other nucleotide sequence analyzer is normally used in nucleotide sequence analysis.

A gene encoding protein (a) or (b) comprises an open reading frame encoding protein (a) or (b) and a termination codon located at the 3' end thereof. In addition, a gene encoding protein (a) or (b) may comprise an untranslated region (UTR) at the 5' end and/or the 3' end of the open reading frame.

An example of a gene encoding protein (a) is a gene comprising DNA comprising nucleotides 103 through 1488 of the nucleotide sequence represented by Seq. ID No. 1. Nucleotides 103 through 1488 of the nucleotide sequence represented by Seq. ID No. 1 here are an open reading frame encoding protein (a), with the translation initiation codon being located in nucleotides 103 through 105 of the nucleotide sequence represented by Seq. ID No. 1 and the termination codon in nucleotides 1489 through 1491. There are no particular limits on the nucleotide sequence of a gene encoding protein (a) as long as it encodes protein (a), and the nucleotide sequence of the open reading frame is not limited to the sequence of nucleotides 103 through 1488 of the nucleotide sequence represented by Seq. ID No. 1.

A gene encoding protein (a) can be obtained by chemical synthesis following the nucleotide sequence. Chemical synthesis of DNA can be accomplished using a commercial DNA synthesizer such as for example a DNA synthesizer using the thiophosphate method (Shimazu) or a DNA synthesizer using the phosphoamidite method (Perkin Elmer).

An example of a gene encoding protein (b) is a gene which hybridizes under stringent conditions with DNA complementary to DNA comprising the sequence of nucleotides 103 through 1488 in the nucleotide sequence represented by Seq. ID No. 1, and which comprises DNA encoding a protein expression of which is selectively elevated in abnormal cells or abnormal tissue (particularly lung cancer cells or lung cancer tissue).

"Stringent conditions" are for example conditions of 42°C, 2 x SSC and 0.1% SDS or preferably 65°C, 0.1 x SSC and 0.1% SDS.

An example of DNA which hybridizes under stringent conditions with DNA complementary to DNA comprising the sequence of nucleotides 103 through 1488 in the nucleotide sequence represented by Seq. ID No. 1 is DNA having at least 50% or greater or preferably 70% or greater or more preferably 90% or greater homology with DNA comprising the sequence of nucleotides 103 through 1488 in the nucleotide sequence represented by Seq. ID No. 1. Specific examples include genes comprising DNA comprising nucleotide sequences in which in the sequence of nucleotides 103 through 1488 in the nucleotide sequence represented by Seq. ID No. 1 the 126^{th} nucleotide a is replaced by nucleotide g, the 128^{th} nucleotide a by nucleotide g, the 130^{th} nucleotide a by nucleotide g, the 139^{th} nucleotide a by nucleotide g, the 175^{th} nucleotide a by nucleotide g, the 179^{th} nucleotide t by nucleotide c, the 188^{th} nucleotide t by nucleotide a, the 216^{th} nucleotide t by nucleotide c, the 329^{th} nucleotide a by nucleotide g, the 348^{th} nucleotide c by nucleotide t, the 349^{th} nucleotide a by nucleotide g, the 370^{th} nucleotide t by nucleotide c, the 414^{th} nucleotide t by nucleotide c, the 434^{th} nucleotide t by nucleotide c, the 436^{th} nucleotide t by nucleotide c, the 449^{th} nucleotide a by nucleotide g, the 484^{th} nucleotide g by nucleotide a, the 535^{th} nucleotide c by nucleotide t, the 653^{rd} nucleotide t by nucleotide c, the 701^{st} nucleotide a by nucleotide g, the 782^{nd} nucleotide a by nucleotide g, the 824^{th} nucleotide a by nucleotide g, the 877^{th} nucleotide t by nucleotide c, the 948^{th} nucleotide t by nucleotide c, the 989^{th} nucleotide a by nucleotide g, the 1025^{th} nucleotide t by nucleotide c, the 1094^{th} nucleotide t by nucleotide c, the 1163^{rd} nucleotide a by nucleotide g, the 1208^{th} nucleotide g by nucleotide a, the 1258^{th} nucleotide a by nucleotide g, the 1300^{th} nucleotide c by nucleotide t, the 1302^{nd} nucleotide c by nucleotide t, the 1316^{th} nucleotide t by nucleotide c, the 1366^{th} nucleotide c by nucleotide t, the 1369^{th} nucleotide t by nucleotide c, or the 1455^{th} nucleotide a by nucleotide g. A gene encoding protein (b) includes a gene having a substitution in one of the aforementioned substitution sites and a gene having substitutions in any two or more sites.

A gene encoding protein (b) is obtained for example by artificially introducing a mutation into a gene encoding protein (a) by a known method such as site-specific mutagenesis. The mutation may be introduced for example using a mutation introduction kit such as a Mutant-K (Takara), Mutant-G (Takara) or a Takara LA PCR in vitro Mutagenesis series kit. A gene the nucleotide sequence of which has already been determined can be obtained by chemical synthesis according to the nucleotide sequence.

Proteins (a) and (b) can be manufactured by expressing the genes encoding the respective proteins in host cells according to the following steps for example. (Preparation of recombinant vector and transformant)

To prepare a recombinant vector, a DNA fragment of a suitable length is prepared which comprises the coding region for the target protein. Alternatively, DNA is prepared with nucleotides replaced in the nucleotide sequence of the coding region for the target protein so that the codons are optimal for expression in the host cells.

A recombinant vector is prepared by inserting this DNA fragment downstream from the promoter of an appropriate expression vector, and this recombinant vector is introduced into appropriate host cells to obtain a transformant capable of producing the target protein. The aforementioned DNA fragment needs to be incorporated into the vector so that its functions can be expressed, and in addition to the promoter the vector may contain enhancers and other cis-elements, splicing signals, poly A addition signals, selection markers (such as the dihydrofolic acid reductase gene, ampicillin resistance gene or neomycin resistance gene), ribosome binding sequences (SD sequences) and the like.

There are no particular limits on the expression vector as long as it is capable of independent replication in the host cells, and for example plasmid vectors, phage vectors, virus vectors and the like can be used. Examples of plasmid vectors include *E. coli*-derived plasmids (such as pRSET, pBR322, pBR325, pUC118, pUC119, pUC18 and pUC19), B. subtilis-derived plasmids (such as pUB110 and pTP5) and yeast-derived plasmids (such as YEp13, YEp24 and YCp50), examples of phage vectors include gamma-phages (such as Charon4A, Charon21A, EMBL3, EMBL4, gamma-gt10, gamma-gt11 and gamma-ZAP), and examples of virus vectors include animal viruses including retroviruses, vaccinia virus and the like and insect viruses such as baculoviruses and the like.

Any of prokaryotic cells, yeasts, animal cells, insect cells, plant cells or the like can be used as the host cells as long as they can express the target gene. Individual animals, plants, silkworms or the like can also be used.

When using bacterial cells as host cells, for example *Escherichia coli* or other *Escherichia, Bacillus subtilis* or other *Bacillus, Pseudomonas putida* or other *Pseudomonas* or *Rhizobium meliloti* or other *Rhizobium* bacteria can be used as the host cells. Specifically, *E. coli* such as *Escherichia coli* XL1-Blue, *Escherichia coli* XL2-blue, *Escherichia coli* DH1, *Escherichia coli* K12, *Escherichia coli* JM109, *Escherichia coli* HB101 or the like or *Bacillus subtilis* such as *Bacillus subtilis* MI114, *Bacillus subtilis* 207-21 or the like can be used. There are no particular limits on the promoter in this case as long as it is capable of expression in *E. coli* or other bacteria, and for example a trp promoter, lac promoter, P_{L} promoter, P_{R} promoter or other *E. coli-* or phage-derived promoter can be used. An artificially designed and modified promoter such as a tac promoter, lac T7 promoter or let I promoter can also be used.

There are no particular limits on the method of introducing the recombinant vector into the bacteria as long as it is a method capable of introducing DNA into bacteria, and for example electroporation or a method using calcium ions or the like can be used.

When using yeasts as host cells, for example *Saccharomyces cerevisiae, Schizosaccharomyces pombe, Pichia pastoris* or the like can be used as the host cells. There are no particular limits on the promoter in this case as long as it can be expressed in yeasts, and for example a gal1 promoter, gal10 promoter, heat shock protein promoter, MFα1 promoter, PHO5 promoter, PGK promoter, GAP promoter, ADH promoter, AOX1 promoter or the like can be used.

There are no particular limits on the method of introducing the recombinant vector into the yeast as long as it is a method capable of introducing DNA into yeast, and for example the electroporation method, spheroplast method, lithium acetate method or the like can be used.

When using animal cells as host cells, for example monkey COS-7 cells, Vero cells, chinese hamster ovary cells (CHO cells), mouse L cells, rat GH3, human FL cells or the like can be used as the host cells. There are no particular limits on the promoter in the case as long as it can be expressed in animal cells, and for example an SRα promoter, SV40 promoter, LTR (long terminal repeat) promoter, CMV promoter, human cytomegalovirus initial gene promoter or the like can be used.

There are no particular limits on the method of introducing the recombinant vector into the animal cells as long as it is a method capable of introducing DNA into animal cells, and for example the electroporation method, calcium phosphate method, lipofection method or the like can be used.

When using insect cells as host cells, for example *Spodoptera frugiperda* ovary cells, *Trichoplusia ni* ovary cells, cultured cells derived from silkworm ovaries or the like can be used as the host cells. Examples of *Spodoptera frugiperda* ovary cells include Sf9, Sf21 and the like, examples of *Trichoplusia ni* ovary cells include High 5, BTI-TN-5B1-4 (Invitrogen) and the like, and examples of cultured cells derived from silkworm ovaries include *Bombyx mori* N4 and the like.

There are no particular limits on the method of introducing the recombinant vector into the insect cells as long as it is a method capable of introducing DNA into insect cells, and for example the calcium phosphate method, lipofection method, electroporation method or the like can be used.

### (Culture of transformant)

A transformant into which has been introduced a recombinant vector having incorporated DNA encoding the target protein is cultured by normal culture methods. Culture of the transformant can be accomplished according to normal methods used in culturing host cells.

For the medium for culturing a transformant obtained as *E. coli,* yeast or other microbial host cells, either a natural or synthetic medium can be used as long as it contains carbon sources, nitrogen sources, inorganic salts and the like which are convertible by the microorganism and is a medium suitable for efficient culture of the transformant.

Glucose, fructose, sucrose, starch and other carbohydrates, acetic acid, propionic acid and other organic acids, and ethanol, propanol and other alcohols can be used as carbon sources. Ammonia, ammonium chloride, ammonium sulfate, ammonium acetate, ammonium phosphate and other ammonium salts of inorganic or organic acids and peptone, meat extract, yeast extract, corn steep liquor, casein hydrolysate and the like can be used as nitrogen sources. Monopotassium phosphate, dipotassium phosphate, magnesium phosphate, magnesium sulfate, sodium chloride, ferrous sulfate, manganese sulfate, copper sulfate, calcium carbonate and the like can be used as inorganic salts.

Culture of a transformant obtained as *E. coli,* yeast or other microbial host cells can be accomplished under aerobic conditions such as a shaking culture, aerated agitation culture or the like. The culture temperature is normally 25 to 37°C, the culture time is normally 12 to 48 hours, and the pH is maintained at 6 to 8 during the culture period. pH can be adjusted using inorganic acids, organic acids, alkaline solution, urea, calcium carbonate, ammonia or the like. Moreover, antibiotics such as ampicillin, tetracycline and the like can be added to the medium as necessary for purposes of culture.

When culturing a microorganism transformed with an expression vector using an inducible promoter as the promoter, an inducer can be added to the medium as necessary. for example, isopropyl-beta-D-thiogalactopyranoside or the like can be added to the medium when culturing a microorganism transformed with an expression vector using a lac promoter, and indoleacrylic acid when culturing a microorganism transformed with an expression vector using a trp promoter.

Commonly used RPMI1640 medium, Eagle's MEM medium, DMEM medium, Ham F12 medium, Ham F12K medium or a medium comprising one of these media with fetal calf serum or the like added can be used as the medium for culturing a transformant obtained with animal cells as the host cells. The transformant is normally cultured for 3 to 10 days at 37°C in the presence of 5% CO₂. Moreover, an antibiotic such as kanamycin, penicillin, streptomycin or the like can be added as necessary to the medium for purposes of culture.

Transformants which can use commonly used TNM-FH medium (Pharmingen), Sf-900 II SFM medium (Gibco-BRL), ExCell400, ExCell405 (JRH Biosciences) or the like as the medium for culturing a transformant obtained with insect cells as the host cells are normally cultured for 3 to 10 days at 27°C. An antibiotic such as gentamicin or the like can be added to the medium as necessary for purposes of culture.

The target protein can also be made to be expressed as a secretory protein or fused protein. Examples of proteins to be fused include beta-galactosidase, protein A, protein A IgG binding region, chloramphenicol acetyltransferase, poly(Arg), poly(Glu), protein G, maltose binding protein, glutathione S transferase, polyhistidine chain (His-tag), S peptide, DNA binding protein domain, Tac antigen, thioredoxin, green fluorescent protein and the like.

### (Isolation and purification of protein)

The target protein is obtained by collecting the target protein from a culture of the transformant. "Culture" here includes culture supernatant, cultured cells, cultured bacterial cells and crushed cells or bacterial cells.

When the target protein accumulates in cells of the transformant, the cells in the culture can be collected by centrifuging the culture and washed and crushed, and the target protein extracted. When the target protein is secreted outside the cells of the transformant, either the supernatant is used as is or cells or bacterial cells are removed from the culture supernatant by centrifugation or the like.

The resulting protein (a) or (b) can be purified by a method such as solvent extraction, salting-out desalting with ammonium sulfate or the like, precipitation with an organic solvent, diethylaminoethyl (DEAE) sepharose, ion exchange chromatography, hydrophobic chromatography, gel filtration, affinity chromatography or the like.

Protein (a) or (b) can also be manufactured by a chemical synthesis method such as the Fmoc (fluorenyl methyloxycarbonyl) method or tBoc (t-butyloxycarbonyl) method based on its amino acid sequence. In this case, a commercial peptide synthesizer can be used.

An antibody or fragment thereof of the present invention is an antibody or fragment thereof capable of reacting to protein (a) or (b). "Antibody" here includes both monoclonal antibodies and polyclonal antibodies, while "monoclonal antibodies and polyclonal antibodies" include all classes of monoclonal antibodies and polyclonal antibodies. "Antibody" also includes antiserum obtained by immunizing a rabbit, mouse or other immune animal with protein (a) or (b), human antibodies, humanized antibodies obtained by gene recombination and the like. A "fragment thereof" includes Fab fragments, F(ab)'₂ fragments, single-chain antibodies (scFv) and the like.

An antibody or fragment thereof of the present invention is prepared by using protein (a) or (b) as an immunizing antigen. For example, (i) crushed cells or tissue or purified crushed cells or tissue expressing protein (a) or (b), (ii) a recombinant protein made to be expressed in *E. coli,* insect cells, animal cells or other host cells by introduction of a gene encoding protein (a) or (b) using gene recombination technology, or (iii) a chemically synthesized peptide or the like can be used as the immunizing antigen.

To prepare polyclonal antibodies, rats, mice, guinea pigs, rabbits, sheep, horses, cows or other mammals are immunized using the immunizing antigen. Mice are used by preference as the immune animals from the standpoint of ease of antibody preparation. For purposes of inducing antibody production during immunization, multiple immunizations are preferably performed using an emulsion prepared with an immune assistant such as Freund's complete adjuvant. In addition to Freund's complete adjuvant (FCA), Freund's incomplete adjuvant (FIA), ammonium hydroxide gel or the like can be used as the immune assistant. The amount of antigen administered per individual mammal can be set appropriately according to the type of mammal, but in the case of a mouse it is normally 50 to 500 µg. Administration is normally intravenous, subcutaneous or intraperitoneal for example. The interval between immunizations is normally a few days to a few weeks, or preferably 4 days to 3 weeks, for a total of 2 to 8 or preferably 2 to 5 immunizations. 3 to 10 days after the final immunization antibody titer to protein (a) or (b) is measured, blood is collected after antibody titer has risen, and antiserum is prepared. Antibody titer is measured by enzyme-linked immunosorbent assay (ELISA), radio-immuno-assay (RIA) or the like.

When antibodies need to be purified from antiserum, a known method such as salting out with ammonium sulfate, gel chromatography, ion exchange chromatography, affinity chromatography or the like or a combination of these can be selected as appropriate.

To prepare monoclonal antibodies, mammals are immunized with an immunizing antigen as in the case of polyclonal antibodies, and antibody-producing cells are collected 2 to 5 days after the final immunization. Examples of antibody-producing cells include spleen cells, lymph node cells, thymocytes, peripheral blood cells and the like, and spleen cells are generally used.

Next, cell fusion of the antibody-producing cells with myeloma cells is performed to obtain a hybridoma. Commonly available strains of cells derived from humans, mice or other mammals can be used as the myeloma cells for fusion with the antibody-producing cells. Preferably the cell strain used is one having drug selectivity which has the property of not surviving in an unfused state in a selection medium (such as HAT medium) but only surviving when fused with antibody-producing cells. Specific examples of myeloma cells include P3X63-Ag.8.U1 (P3U1), P3/NSI/1-Ag4-1, Sp2/0-Ag14 and other mouse myeloma cell strains.

For cell fusion, the antibody-producing cells and myeloma cells are mixed at a specific ratio (such as 1:1 to 1:10) in an animal cell culture medium such as DMEM or RPMI-1640 medium or the like containing no serum, and a fusion reaction is performed in the presence of a cell fusion promoter such as polyethylene glycol or by an electrical pulse method (such as electroporation).

After cell fusion treatment the cells are cultured using a selection medium to select the target hybridoma. Next, the culture supernatant of the proliferated hybridoma is screened for the presence or absence of the target antibodies. The hybridoma can be screened by ordinary methods, with no particular limitations. For example, part of the culture supernatant contained in a well grown as the hybridoma can be collected and screened by enzyme-linked immunosorbent assay (ELISA), radio-immuno-assay (RIA) or the like.

The hybridoma can be cloned for example by limiting dilution analysis, soft agar cloning, fibrin gel cloning, fluorescence activated cell sorting or the like. Ultimately a hybridoma producing monoclonal antibodies is obtained.

An ordinary cell culture method or the like can be used as the method for collecting monoclonal antibodies from the resulting hybridoma. In the cell culture method, if for example the hybridoma is cultured for 3 to 10 days under ordinary culture conditions (for example, 37°C, 5% CO₂ concentration) in an animal cell culture medium such as MEM medium or RPMI-1640 medium containing 10 to 20% fetal calf serum, monoclonal antibodies can be obtained from the culture supernatant. Alternatively, the hybridoma can be transplanted intraperitoneally into mice, ascites collected after 10 to 14 days, and monoclonal antibodies obtained from the ascites.

When monoclonal antibodies need to be purified, a known method such as salting out with ammonium sulfate, gel chromatography, ion exchange chromatography, affinity chromatography or the like or a combination of these can be selected as appropriate.

When monoclonal antibodies are used with the object of administration to human beings (antibody therapy), human antibodies or humanized antibodies should be used to decrease immunogenicity. Human antibodies or humanized antibodies can be obtained for example by preparing a hybridoma using mice or the like having introduced human antibody genes as the immune animals, or by using a library of antibodies presented on phages. Specifically, a transgenic animal having a repertory of human antibody genes can be immunized with the antigenic protein, protein-expressing cells or a lysate thereof to obtain antibody-producing cells which are fused with myeloma cells to produce a hybridoma which is used to obtain human antibodies to the target protein (see International Patent Applications Nos. WO92-03918, WO93-2227, WO94-02602, WO96-33735 and WO96-34096). Alternatively, phages presenting antibodies which bind to the antigenic protein, protein-expressing cells or a lysate thereof can be screened from an antibody library of several different human scFv's presented on phages to select the scFv which binds to the target protein (Griffiths et al., *EMBO J.* 12, 725-734, 1993).

The diagnostic method of the present invention comprises a step wherein the level of a gene encoding protein (a) or (b) expressed in a sample collected from a test animal is used as the marker to diagnose whether or not the test animal suffers from a disease involving elevated expression of that gene. Since expression of a gene encoding protein (a) or (b) is elevated only in abnormal cells and abnormal tissue and not in normal cells or normal tissue, the level of expression of that gene can be used as a marker for diagnosing diseases involving elevated expression of that gene.

There are no particular limits on the test animal, which may be a human, monkey, cow, sheep, goat, horse, pig, rabbit, dog, cat, rat, mouse or other mammal for example. There are also no particular limits on the specimen collected from the test animal, and for example blood, serum or the like can be used as well as tissue or organs which are the object of diagnosis. There are no particular limits on the tissue or organs which are the object of diagnosis, and examples include the brain, hypophysis, spinal cord, salivary glands, thymus, thyroid gland, lungs, breasts, skin, skeletal muscle, heart, liver, spleen, adrenal gland, pancreas, stomach, small intestine, large intestine, rectum, bladder, prostate gland, testes, ovaries, placenta, uterus, bone marrow, peripheral monocytes and the like.

"The level of expression of a gene encoding protein (a) or (b)" includes the level of transcription into mRNA of a gene encoding protein (a) or (b) and the level of translation into protein (a) or (b). Consequently, the level of expression of a gene encoding protein (a) or (b) in a specimen can be measured based on the amount of mRNA encoding protein (a) or (b) present in the specimen or the amount of protein (a) or (b) present in the specimen.

A known genetic analysis technique such as a hybridization technique (for example, the northern hybridization, dot blotting or DNA micro-array method or the like), gene amplification technique (for example, RT-PCR or the like) can be used to measure the amount of mRNA encoding protein (a) or (b) present in a sample.

When using a hybridization technique, an oligonucleotide or polynucleotide capable of hybridizing with a nucleic acid encoding protein (a) or (b) can be used as the probe, while when using a gene amplification technique such an oligonucleotide or polynucleotide can be used as the primer.

"A nucleic acid encoding protein (a) or (b)" encompasses both DNA and RNA, including for example mRNA, cDNA, cRNA and the like. The nucleotides making up the oligonucleotide or polynucleotide may be either deoxyribonucleotides or ribonucleotides. There are no particular limits on the nucleotide length of the oligonucleotide, which is normally 15 to 100 nucleotides or preferably 18 to 30 nucleotides. There are also no particular limits on the nucleotide length of the polynucleotide, which is normally 50 to 1000 nucleotides or preferably 200 to 800 nucleotides.

An oligonucleotide or polynucleotide capable of hybridizing with a nucleic acid encoding protein (a) or (b) is preferably one capable of hybridizing specifically with a nucleic acid encoding protein (a) or (b). "Capable of hybridizing specifically" means capable of hybridizing under stringent conditions, and "stringent conditions" are for example conditions of 42°C, 2 X SSC and 0.1% SDS or preferably 65°C, 0.1 X SSC and 0.1% SDS.

The nucleotide sequence of an oligonucleotide or polynucleotide capable of hybridizing with a nucleic acid encoding protein (a) or (b) can be designed based on the nucleotide sequence of a nucleic acid encoding protein (a) or (b). The oligonucleotide or polynucleotide is designed for example so as to be capable of hybridizing with the CDS region of a nucleic acid encoding protein (a) or (b), so as to be capable of hybridizing with a region at the 5' end or 3' end of the CDS region, or so as to be capable of hybridizing with a region extending from CDS region to a region at the 5' or 3' end thereof. A restriction enzyme recognition sequence, tag or the like can be added to the 5' end of the primer, and a fluorescent dye, radioisotope or other label can be added to the primer and probe.

RT-PCR is used as an example of a specific method for measuring the amount of mRNA encoding protein (a) or (b) present in a specimen. Total RNA is extracted from a specimen collected from a test animal, cDNA is synthesized from the extracted total RNA, the synthesized cDNA is used as the template for PCR using a primer capable of hybridizing with cDNA encoding protein (a) or (b), and the amount of mRNA encoding protein (a) or (b) can be measured by assaying the PCR amplified fragments. In this case, PCR is performed under conditions in which the amount of PCR amplified fragments produced reflects the amount of the initial template cDNA (for example, a number of PCR cycles at which the PCR amplified fragments increase exponentially).

There are no particular limits on the method of assaying the PCR amplified fragments, and PCR amplified fragments can be assayed for example by an assay method using radioisotopes (RI) or an assay method using a fluorescent dye.

Examples of assay methods using RI include (i) a method in which an RI-labeled nucleotide (for example, ³²P-labeled dCTP or the like) is added as a substrate to a reaction liquid and incorporated into the PCR amplified fragments to RI label the PCR amplified fragments, the PCR amplified fragments are isolated by electrophoresis or the like, and radioactivity is measured to assay the PCR amplified fragments, (ii) a method in which PCR amplified fragments are RI labeled using an RI labeled primer, the PCR amplified fragments are isolated by electrophoresis and the radioactivity is measured to assay the PCR amplified fragments and (iii) a method in which the PCR amplified fragments are first subjected to electrophoresis and blotted on a membrane, an RI-labeled probe is hybridized and radioactivity is measured to assay the PCR amplified fragments. Radioactivity can be measured for example using a liquid scintillation counter, X-ray film, imaging plates or the like.

Examples of assay methods using fluorescent dyes include (i) a method in which PCR amplified fragments are dyed using a fluorescent dye intercalating with duplex DNA (for example, ethidium bromide (EtBr), SYBR Green I, PicoGreen or the like), and the strength of fluorescence resulting from illumination with excitation light is measured to assay the PCR amplified fragments and (ii) a method in which PCR amplified fragment are labeled with fluorescent dye using a primer labeled with fluorescent dye, the PCR amplified fragments are isolated by electrophoresis and the fluorescent strength is measured to assay the PCR amplified fragments. The fluorescent strength can be measured using a CCD camera, fluorescence scanner, spectrofluorometer or the like.

A known protein analysis technique such as western blotting using antibodies or fragments thereof capable of reacting to protein (a) or (b), immune precipitation, ELISA, tissue immunoblotting or the like can be used to measure the amount of protein (a) or (b) present in a specimen.

Radio immunoassay (RIA), enzyme immunoassay (EIA), chemiluminescence immunoassay (CLIA), fluorescence immunoassay (FIA), tissue immunoblotting or the like for example can be used in measuring the amount of protein (a) or (b) in a specimen using antibodies or fragments thereof capable of reacting with protein (a) or (b). Specifically, using a solid-phase carrier (for example, an immunoplate, latex particles or the like) on which antibodies are bound by physical adsorption, chemical binding or the like, protein (a) or (b) in the specimen is first supplemented, and the supplemented protein (a) or (b) can then be assayed using labeled antibodies (for example, antibodies labeled with peroxidase, alkaline phosphatase or another enzyme or fluorescence, umbelliferone or another fluorescent substance or the like) having a different antigen recognition site for protein (a) or (b) than the antibodies fixed on the solid-phase carrier.

The amount of protein (a) or (b) present in a specimen can also be measured by measuring the activity of protein (a) or (b) in the specimen. The activity of protein (a) or (b) can be measured by a known method such as ELISA, western blotting or the like using antibodies or fragments thereof capable of reacting to protein (a) or (b).

The measurement values for level of expression of a gene encoding protein (a) or (b) are preferably corrected based on the measurement values for level of expression of a gene encoding a protein (such as beta-actin or GAPDH) the expressed level of which does not fluctuate greatly.

In the diagnostic method of the present invention, a test animal can be diagnosed as suffering from a disease involving elevated expression of a gene encoding protein (a) or (b) when the level of expression of that gene in a sample collected from a test animal is higher than the level of expression of that gene in a specimen collected from a normal animal.

When comparing the levels of expression of a gene encoding protein (a) or (b) in a test animal and a normal animal, the same types of cells or tissues (including organs) are used as the specimens. Moreover, when comparing the levels of expression of a gene encoding protein (a) or (b) in a test animal and a normal animal it is desirable to assay the levels of expression of the gene encoding protein (a) or (b) in multiple normal animals (normal animal group), set the normal range from the distribution of those values, and evaluate from this whether the level of expression of the gene encoding protein (a) or (b) in the test animal is above or below the normal range. In this case, if the level of expression of the gene in a specimen from a test animal is above the normal range, the test animal can be diagnosed as suffering from the disease.

The diagnostic method of the present invention can be used for diseases involving elevated expression of a gene encoding protein (a) or (b), with no particular limits on the type of disease that can be diagnosed. "Involving elevated expression of a gene" here includes both cases in which the disease occurs because expression of the gene is elevated and cases in which expression of the gene is elevated because of the presence of the disease.

Examples of diseases which can be diagnosed by the diagnostic method of the present invention include lung cancers, and examples of lung cancers include pulmonary adenocarcinoma, pulmonary squamous cell carcinoma, large cell lung cancer, small cell lung cancer and the like. Of these cancers, the diagnostic method of the present invention is particular useful in the diagnosis of pulmonary adenocarcinoma. Moreover, seeing that the gene expression profiles of primary lung cancer tissues tend to differ from those of lung cancer tissues which have metastasized from large intestinal cancer, stomach cancer or the like (Arindam Bhattacharjee et al., *PNAS* Vol. 98, 13790-13795, 2001), the diagnostic method of the present invention is particularly useful for diagnosing primary lung cancers. Lung cells, lung tissue, blood, serum and the like collected as samples from test animals are usually used in diagnosing lung cancer, but since in some cases expression of a gene encoding protein (a) or (b) is elevated in tissues or organs to which lung cancer cells have metastasized, lung cancer can also be diagnosed using tissues or organs other than the lungs. However, when tissues or organs other than the lungs are used it is impossible to determine whether elevated expression of a gene encoding protein (a) or (b) is due to independent abnormalities of tissues or organs other than the lungs or to metastasis of lung cancer cells, so it is only possible to diagnosis that lung cancer is one of the diseases from which the test animal may be suffering.

The diagnostic kit of the present invention comprises an oligonucleotide or polynucleotide capable of hybridizing with a nucleic acid encoding protein (a) or (b), or else an antibody or fragment thereof capable of reacting to protein (a) or (b). This oligonucleotide or polynucleotide or antibody or fragment thereof is included in the diagnostic kit of the present invention as a reagent for measuring the level of expression of a gene encoding protein (a) or (b) in a sample collected from a test animal, and using the diagnostic kit of the present invention it is possible to diagnose whether or not a test animal suffers from a disease involving elevated expression of that gene.

The diagnostic kit of the present invention can be in any form and may comprise any reagents, tools or the like as long as it comprises the aforementioned oligonucleotide or polynucleotide or the aforementioned antibody or fragment thereof.

When the diagnostic kit of the present invention comprises the aforementioned oligonucleotide or polynucleotide, it can comprise one or two or more kinds of reagents necessary for PCR (such as H₂O, buffer, MgCl₂, dNTP mix, Taq polymerase and the like), reagents necessary for assaying PCR amplified fragments (such as RI, fluorescent dye and the like), DNA microarrays, DNA chips and the like.

Moreover, when the diagnostic kit of the present invention comprises the aforementioned antibody or fragment thereof, it can comprise one or two or more kinds of solid-phase carriers for fixing the antibody or fragment thereof (such as immunoplates, latex particles and the like), anti-gamma-globulin antibodies (secondary antibodies), labels for the antibodies (including secondary antibodies) and fragments thereof (such as enzymes, fluorescent substances and the like), various reagents (such as enzyme substrates, buffers, diluents, etc.) and the like.

The screening method of the present invention comprises a step in which a reduction effect on the level of expression of a gene encoding protein (a) or (b) in cells or tissue in which the level of expression of a gene encoding protein (a) or (b) is elevated is used as the marker for evaluating the preventative and therapeutic effects of a target substance on a disease involving elevated expression of that gene. In the screening method of the present invention, preventative and therapeutic substances for diseases involving elevated expression of a gene encoding protein (a) or (b) can be screened by selecting substances having a reduction effect on the level of expression of that gene.

The screening method of the present invention can be widely used for screening substances for preventing and treating diseases involving elevated expression of a gene encoding protein (a) or (b), and there are no particular limits on the target disease. Examples of the target disease for the screening method of the present invention include lung cancers, and examples of lung cancers include pulmonary adenocarcinoma, pulmonary squamous cell carcinoma, large cell lung cancer, small cell lung cancer and the like. Of these lung cancers, the screening method of the present invention is useful for screening substances having preventative and therapeutic effects against pulmonary adenocarcinoma in particular.

In the screening method of the present invention, "a reduction effect on the level of expression of a gene encoding protein (a) or (b)" includes effects against any of the steps of transcription and translation of a gene encoding protein (a) or (b) and active expression and the like of protein (a) or (b).

The screening method of the present invention can be performed either in vivo or in vitro.

In vivo, for example, preventative and therapeutic substances for diseases involving elevated expression of a gene encoding protein (a) or (b) can be screened by evaluating the preventative and therapeutic effects of candidate substances against such diseases using as the marker the reduction effect on the level of expresion of that gene after administration of candidate substance when a candidate substance is first administered to a model animal in which the level of expression of a gene encoding protein (a) or (b) is elevated, a specimen (cells or tissue (including organs) in which the level of expression of the gene was elevated before administration of the candidate substance) is collected from the model animal, and the level of expression of the gene in the specimen is measured.

Examples of model animals to be used for screening in vivo include humans, cows, sheep, goats, horses, pigs, rabbits, dogs, cats, rats, mice and other mammals. Transgenic animals in which the level of expression of a gene encoding protein (a) or (b) has been artificially elevated can also be used as model animals for administration of candidate substances. Such transgenic animals can be obtained for example by a known method such as (i) a method of mixing an egg with a gene encoding protein (a) and (b) and treating it with calcium phosphate, (ii) a method of directly inserting a gene encoding protein (a) or (b) into the nucleus of a pronuclear egg under a phase contrast microscope, or (iii) a method using embryonic stem cells (ES cells). Elevation of the expression level of a gene encoding protein (a) or (b) in a transgenic mammal includes forced expression when the gene is introduced as an exogenous gene, elevation of the expression level of the host's own gene, and suppression of degradation of protein (a) or (b).

In vitro, preventative and therapeutic substances for diseases involving elevated expression of a gene encoding protein (a) or (b) can be screened based on an evaluation of the preventative and therapeutic effects of candidate substances on such diseases using as the marker the reduction effect on the level of expression of the gene after contact with a candidate substance for example when a candidate substance is brought into contact with cells or tissue (including organs) in which the level of expression of the gene encoding protein (a) or (b) is elevated, and the expression level of the gene in the cells or tissue is measured.

Cells which can be used for screening in vitro include cell strains derived from humans, monkeys, mice, rats and the like for example. Moreover, cells in which the level of expression of a gene encoding protein (a) or (b) has been artificially elevated can also be used for in vitro screening. Such cells can be obtained by inserting a gene encoding protein (a) or (b) into a suitable expression vector and introducing that vector into suitable host cells.

The screening kit of the present invention comprises an oligonucleotide or polynucleotide capable of hybridizing with a nucleic acid encoding protein (a) or (b) or an antibody or fragment thereof capable of reacting to protein (a) or (b). This oligonucleotide or polynucleotide or antibody or fragment thereof is including in the screening kit of the present invention as a reagent for measuring the level of expression of a gene encoding protein (a) or (b) in a specimen collected from a test animal, and preventative and therapeutic substances for diseases involving elevated expression of that gene can be screened using the screening kit of the present invention.

The screening kit of the present invention can be in any form as long as it comprises the aforementioned oligonucleotide or polynucleotide of the aforementioned antibody or fragment thereof, and may comprise in addition to the various reagents and tools listed for the aforementioned diagnostic kit candidate substances, candidate substance synthesis kits, model animal rearing kits and the like.

The present invention is explained in detail below with reference to examples, but the present invention is not limited by these. Gene manipulation using *E. coli* and the like was performed in principle according to the methods described in Molecular Cloning (Cold Spring Harbor Lab. Press, 1989).

### (Example 1) Identification of gene which is specifically expressed in human pulmonary adenocarcinoma tissue

Expression analysis of mRNA in extracted human pulmonary adenocarcinoma tissue was performed using a GeneChip (Gene Chip™ HG-133 A,B Target; Affymetryx) in order to identify a gene which is specifically expressed in pulmonary adenocarcinoma tissue.

### (1) Gene expression analysis of human pulmonary adenocarcinoma tissue and human normal lung tissue

First, total RNA was prepared using ISOGEN (Nihon Gene) according to the enclosed methods from tumor sites of pulmonary adenocarcinoma tissue comprising various stages and degrees of differentiation (12 cases) and from normal lungs (1 case) (see Table 1). Next, mRNA expression in pulmonary adenocarcinoma and normal lungs was analyzed using a pulmonary adenocarcinoma GeneChip™ HG-U133A,B (Affymetryx). That is, using as the samples 5µg of a mixture of equal amounts of total RNA prepared from tumor sites in 12 cases and 5µg of total RNA prepared from a normal lung in 1 case, gene expression analysis was performed according to the Expression Analysis Technical Manual (Affymetryx). The expressed amount of each gene was calculated as a relative value given a mean value of 100 for the expression scores of all genes in each analysis.

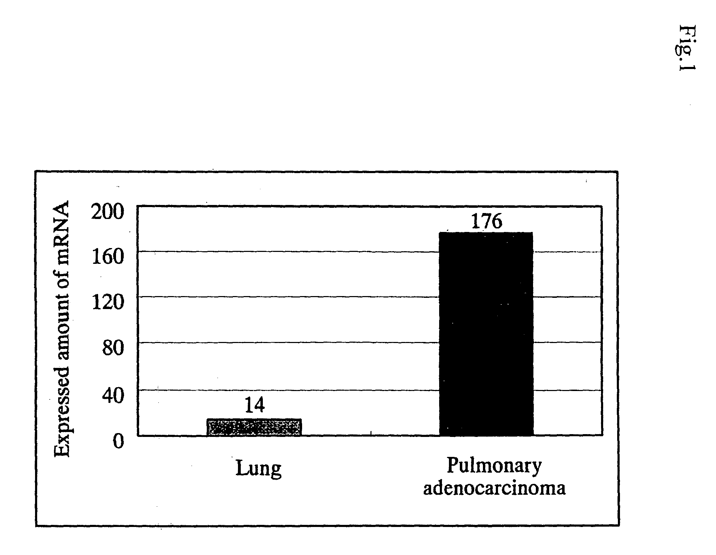

As a result, as shown in Figure 1, the amount of mRNA reacting with the 230349_at_u133B probe which was expressed in pulmonary adenocarcinoma tissue was 12.6 times the expressed amount of the mRNA in a normal lung.

**(Table 1)**

| Organ | Origin | Lot Number |
|---|---|---|
| Brain | Clontech # 64020-1 | 101041 |
| Fetal brain | Clontech #64094-1 | 2020902 |
| Hypophysis | Clontech #6584-1 | 2010981 |
| Spinal cord | Clontech #6593-1 | 111062 |
| Salivary gland | Clontech #64026-1 | 1011322 |
| Thymus | Ambion #7964 | 101P0101A |
| Thyroid gland | Stratagene #735040 | 510225 |
| Trachea | Clontech #64091-1 | 1010201 |
| Lung | prepared from removed lung | NL_1 |
| Breast | Stratagene #735044 | 610327 |
| Skin | Stratagene #735031 | 120484 |
| Skeletal muscle | Ambion #7982 | 091P0101C |
| Heart | Ambion #7966 | 110P43B |
| Liver | prepared from removed liver | N4 |
| Fetal liver | CHEMICON #356 | 21060678 |
| Spleen | Ambion #7970 | 061P18A |
| Kidney | Ambion #7976 | 071P04B |
| Adrenal gland | Clontech #64096-1 | 2020671 |
| Pancreas | Ambion #7954 | 091P0104A |
| Stomach | prepared from removed stomach | MN15 |
| Small intestine | Ambion #7984 | 091P0201A |
| Large intestine | Ambion #7986 | 071P10B |
| Bladder | Ambion #7990 | 81P0101A |
| Prostate | Ambion #7988 | 081P0103A |
| Testes | Clontech #64027-1 | 6120257 |
| Ovaries | Ambion #7974 | 051P42A |
| Placenta | Ambion #7950 | 061P33B |
| Uterus | Stratagene #735042 | 1100640 |
| Bone marrow | Clontech #64106-1 | 1110932 |
| Peripheral monocytes | prepared from peripheral monocytes | |
| HUVEC | prepared from HUVEC | |

### (2) Expression analysis of normal human tissue

Next, the amount of mRNA reacting with the 230349_at_u133B probe which was expressed in normal human tissues other than lung tissue was analyzed using a Gene chip. The various organs shown in Table 1 were used as the normal human tissues. Using 10 ng of human organ-derived RNA for each sample, gene expression analysis was performed as above. Relative values were calculated given a mean value of 100 as the expression score for all genes.

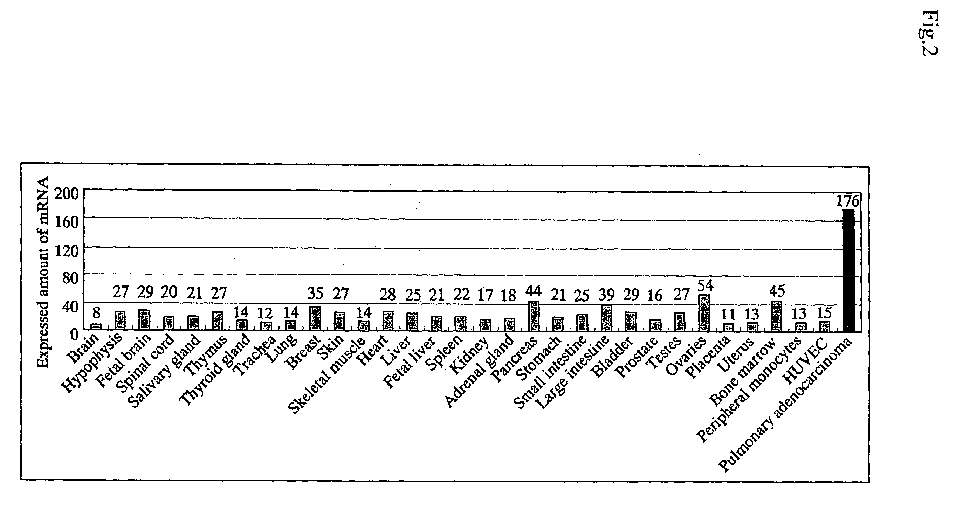

As a result, as shown in Figure 2, the values were low in all normal human tissues as they were in normal lungs. Consequently, it is clear that expression of mRNA which reacts with the 230349_at_u133B probe is specifically elevated in pulmonary adenocarcinoma tissue.

### (Example 2) Isolation and analysis of full-length cDNA

Full-length cDNA was isolated using the RACE method (Rapid amplification cDNA ends) based on the sequence information for 230349_at_u133B.

The target sequence of 230349_at_u133B is human EST (GenBank Accession No. AA213814), but since part of the nucleotide sequence of this human EST (GenBank Accession No. AA213814) has not been identified, the PCR primers GSP1 (Seq. ID No. 3), GSP2 (Seq. ID No. 4) and GSP3 (Seq. ID No. 5) for use in cDNA isolation were designed based on sequence information for an X chromosome comprising this human EST (GenBank Accession No. AA213814), and 5' and 3' cDNA of the target sequence of the probe was amplified using a SMART™ RACE cDNA Amplification kit (Clontech).

Single-strand cDNA was synthesized based on about 400 ng of a mixture of total RNA prepared from the aforementioned pulmonary adenocarcinoma tissue in three cases according to the methods included with the kit, and 5' cDNA was then amplified using the PCR primers GSP1 (Seq. ID No. 3) and GSP2 (Seq. ID No. 4). That is, a PCR reaction was performed according to the methods included with the kit using 1.25 µL of single-stranded cDNA as the template DNA and 5 pmoles of GSP1 (Seq. ID No. 3) or GSP2 (Seq. ID No. 4) as the PCR primer. PCR was performed as 5 cycles of a reaction consisting of a cycle of 5 seconds at 94°C followed by 3 minutes at 72°C, followed by 5 cycles of a reaction consisting of a cycle of 5 seconds at 94°C, 10 seconds at 70°C and 3 minutes at 72°C, and finally by 25 cycles of a reaction consisting of a cycle of 5 seconds at 94°C, 10 seconds at 68°C and 3 minutes at 72°C.

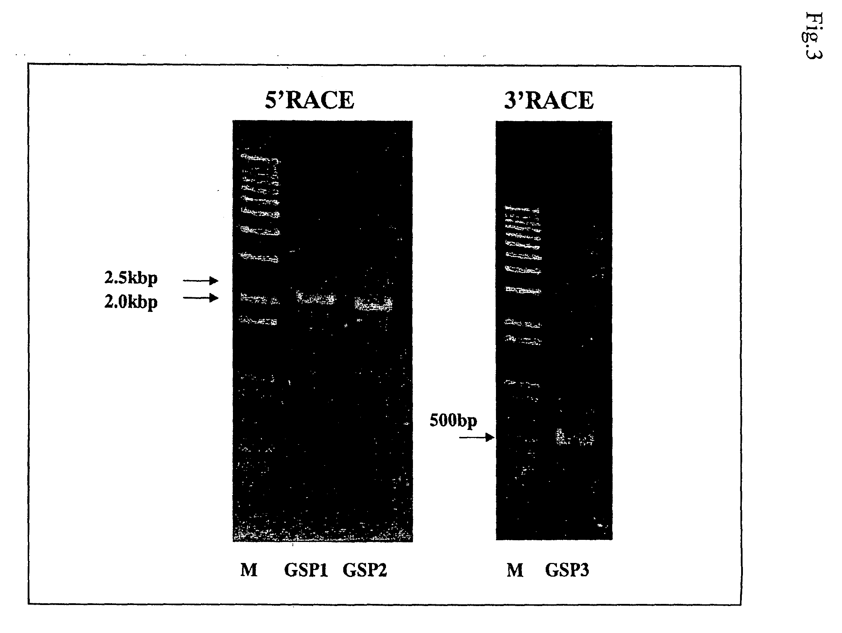

As shown in Figure 3, an important band of about 2000 bp and a band of about 2500 bp were amplified as a result of the aforementioned PCR. Figure 3 shows the results of electrophoresis of the PCR product (1% agarose electrophoresis followed by ethidium bromide staining), with M indicating the molecular weight marker in Figure 3 (1 kbp plus DNA Ladder (Invitrogen)). The amplified product of this PCR reaction was inserted into a pGEM-Teasy vector (Promega) and *E. coli* DH5α (Toyobo) was transformed by ordinary methods, after which plasmid DNA was prepared from the resulting transformant.

Because gene sequences with several differing nucleotides were obtained when the nucleotide sequence of the roughly 2000 bp inserted plasmid DNA gene was first analyzed, the consensus clone was named gene #15, the entire nucleotide sequence of which is represented by Seq. ID No. 1. The nucleotide sequence thought to be the open reading frame of gene #15 is the sequence of nucleotides 103 through 14988 in Seq. ID No. 1, and the amino acid sequence encoded by this open reading frame is represented by Seq. ID No. 2. Moreover, a list of the mutation sites for each clone as discovered by a comparison of gene #15 with each clone having several different nucleotides is shown in Table 2. The roughly 2500 bp amplification product comprises a nucleotide sequence the 5' UTR of which extends further upstream from gene #15, and does not comprise a coding region. The nucleotide sequence of this 5' UTR region is represented by Seq. ID No. 13. In the nucleotide sequence represented by Seq. ID No. 13, the nucleotide sequence up to nucleotide 472 is the nucleotide sequence of the 5' UTR, while the nucleotide sequence beginning at nucleotide 473 is the nucleotide sequence of the coding region (identical to the nucleotide sequence of the coding region of gene #15).

**(Table 2)**

| clone | nucleotide positions | consensus nucleotide sequence | variant nucleotide sequence | consensus amino acid sequence | variant amino acid sequence |
|---|---|---|---|---|---|
| A | 37 | A | G | Thr | Ala |
| | 722 | A | G | Tyr | Cys |
| | 312 | T | C | Asp | Asp |
| B | 332 | T | C | Leu | Pro |
| | 846 | T | C | Ala | Ala |
| C | 26 | A | G | Glu | Gly |
| | 1214 | T | C | Leu | Pro |
| D | 433 | C | T | Pro | Ser |
| | 680 | A | G | Tyr | Cys |
| | 227 | A | G | Glu | Gly |
| E | 551 | T | C | Met | Thr |
| | 1200 | C | T | Leu | Leu |
| | 1353 | A | G | Pro | Pro |
| | 247 | A | G | Thr | Ala |
| F | 347 | A | G | His | Arg |
| | 887 | A | G | Glu | Gly |
| G | 24 | A | G | Ser | Ser |
| | 334 | T | C | Ser | Pro |
| | 246 | C | T | Tyr | Tyr |
| H | 268 | T | C | Ser | Pro |
| | 1156 | A | G | Lys | Glu |
| I | 1061 | A | G | Asp | Gly |
| | 1264 | C | T | Arg | Cys |
| J | 599 | A | G | Gln | Arg |
| | 1106 | G | A | Arg | Lys |
| | 77 | T | C | Val | Ala |
| K | 923 | T | C | Met | Thr |
| | 1198 | C | T | Leu | Phe |
| | 28 | A | G | Arg | Gly |
| | 86 | T | A | Val | Asp |
| L | 114 | T | C | Arg | Arg |
| | 300 | T | - | Phe | all following |
| | 992 | T | C | Leu | amino acids |
| | | | | | are variant |
| | 382 | G | A | Glu | Lys |
| M | 775 | T | C | Trp | Arg |
| | 1267 | T | C | Ser | Pro |
| N | 73 | A | G | Asn | Asp |

The "nucleotide positions" in Table 2 are numbered beginning with 1 as the A of the initiation codon of gene #15 (Seq. ID No. 1). The underlined amino acids are those which do not change in type due to changes in the nucleotide sequence.

Next, the 3' cDNA was isolated as above using a SMART™ RACE cDNA Amplification kit (Clontech) based on the target sequence of the probe. That is, based on a mixture of total RNA prepared from tissue derived from pulmonary adenocarcinoma patients in three cases, single-strand cDNA was synthesized according to the methods included with the kit. Next, the cDNA was amplified using 1.25 µL of single-strand cDNA as the template DNA and 5 pmole of GSP3 (Seq. ID No. 5) as the PCR primer. The PCR reaction was as described above.

As shown in Figure 3, a roughly 500 bp band was amplified as a result of PCR. The PCR product was inserted into a pGEM-T easy vector (Promega) as above, and the nucleotide sequence determined. Of the nucleotide sequence represented by Seq. ID No. 1, the nucleotide sequence beginning with the GSP3 sequence indicates this region.

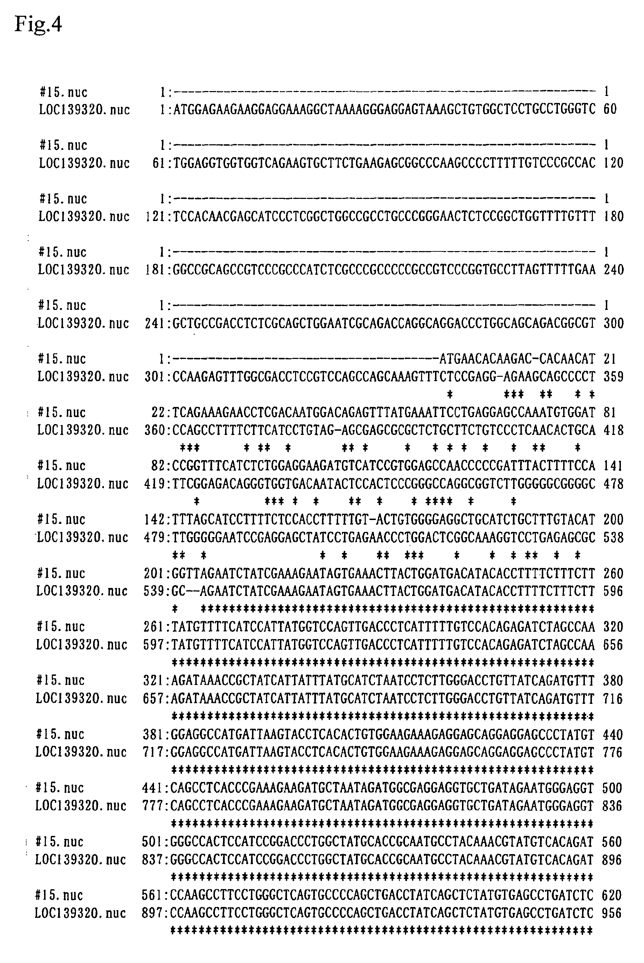

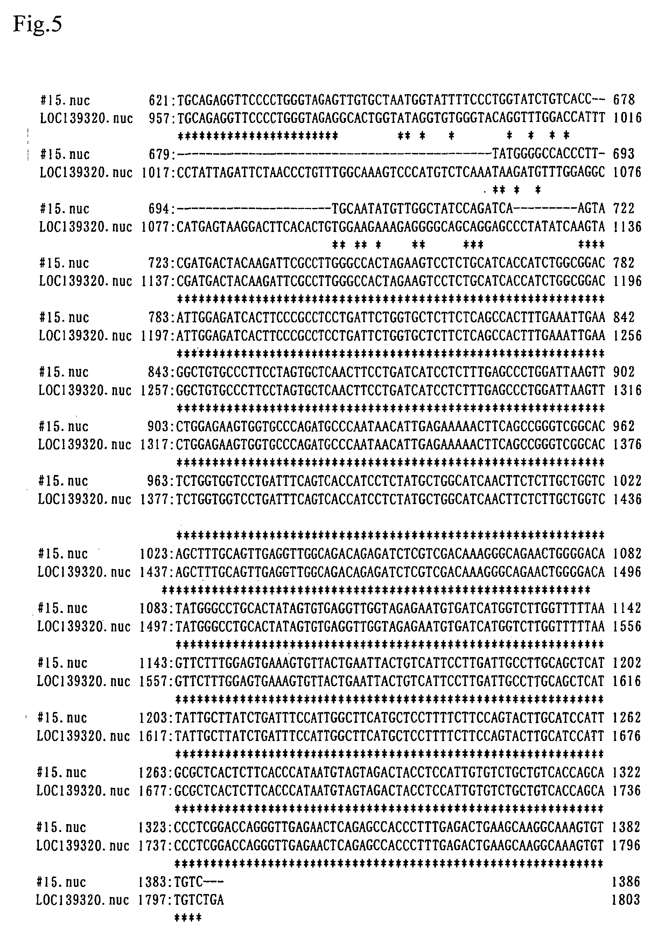

When homologous genes were searched by the Blast method based on the total cDNA sequence obtained by the RACE method above, LOC139320 (GenBank Accession No. XM_066619) was discovered. The results of alignment between the nucleotide sequences of gene #15 and LOC139320 are shown in Figures 4 and 5. As shown in Figures 4 and 5, although some regions of the nucleotide sequences of LOC139320 and the gene #15 isolated and identified in this case matched perfectly, the sequences of the 5' region and central region were different. While LOC139320 is present in a similar X chromosome (Xq22.1) as the target sequence of 230349_at_u133B and parts of the nucleotide sequence match perfectly, it is a nucleotide sequence predicted from the human genome sequence.

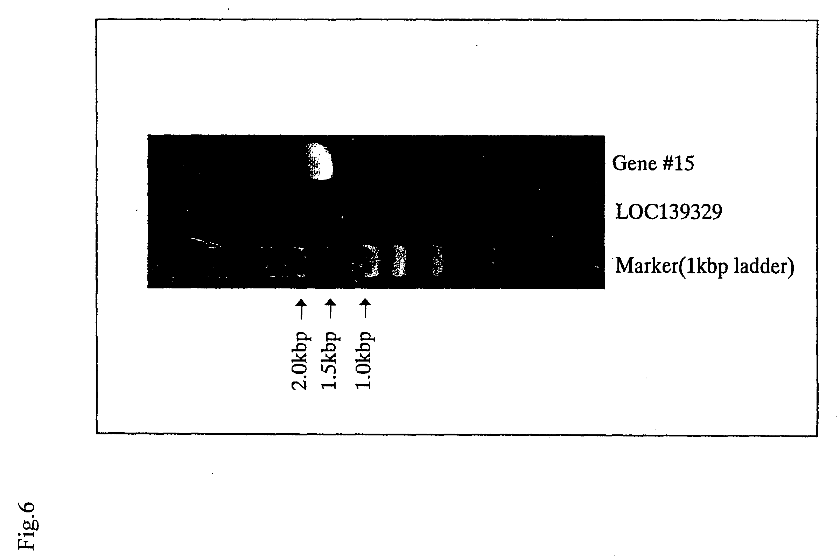

Therefore, in order to determine whether the mRNA which was found in the current mRNA expression analysis to be expressed specifically in pulmonary adenocarcinoma is derived from gene #15 or from LOC139320, PCR primers were designed for the respective regions thought to be the 5' ends, and the presence or absence of expression of mRNA in pulmonary adenocarcinoma tissue was investigated by PCR. That is, a PCR primer for the 5' transcription initiation region of gene #15 (Seq. ID No. 6), a PCR primer for the 5' transcription initiation region of LOC139320 (Seq. ID No. 7) and a common PCR primer for the 3' region (Seq. ID No. 8) were designed and a PCR reaction performed under the same conditions as above using a 5' RACE pulmonary adenocarcinoma cDNA library as the template. As a result, as shown in Figure 6, only DNA fragments derived from gene #15 in pulmonary adenocarcinoma tissue were amplified.

As shown above, we succeeded in identifying the novel gene #15 expression of which is specifically elevated in pulmonary adenocarcinoma tissue.

### (Example 3) Gene expression analysis of gene #15

Expression analysis was performed by RT-PCR and GeneChip (Gene Chip™ HG-133B Target; Affymetryx) to confirm expression of mRNA derived from gene #15.

### (1) Expression analysis of gene #15 in pulmonary adenocarcinoma tissue

Expression of gene #15 in pulmonary adenocarcinoma tissue (12 cases) and normal lung tissue (4 cases) was compared. The sequence of nucleotides 1214 through 1238 in Seq. ID No. 1 was designed as sense direction PCR primer #15_RF (Seq. ID No. 9), while the sequence of nucleotides 1402 through 1378 in Seq. ID No. 1 was designed as antisense direction PCR primer #15_RR (Seq. ID No. 10). Total RNA prepared during Gene chip analysis was used for the pulmonary adenocarcinoma tissue (12 cases), and total RNA prepared by methods similar to those above was used for the normal lung tissue (4 cases) (prepared from extracted lungs). PCR reactions were performed using as the DNA templates single-strand cDNA synthesized from total RNA using reverse transcriptase Superscript II (Gibco BRL), and the amount of mRNA expressed in each tissue was compared.

Each 25 µL of PCR reaction liquid was prepared so as to comprise 500 mM KCl, 100 mM Tris-HCl (pH 8.3), 20 mM MgCl₂, 0.1% Gelatin, 1.25 mM of each dNTP (dATP, dCTP, dGTP, dTTP), 1 µL of single-strand cDNA, 5 pmole each of the sense primer #15_RF (Seq. ID No. 9) and the antisense primer #15_RR (Seq. ID No. 10) and 0.25 µL of recombinant Taq polymerase Mix (FG Pluthero, "Rapid Purification of high-activity Taq DNA polymerase", *Nucl. Acids Res.* 1993 21: 4850-4851), and first subjected to primary denaturing for 3 minutes at 94°C, followed by 30 cycles each consisting of 15 seconds at 94°C, 15 seconds at 57°C and 30 seconds at 72°C. The expressed amount of the human beta-actin gene in each individual RNA was also analyzed as above using a human beta-actin-specific sense primer (Seq. ID No. 11) and antisense primer (Seq. ID No. 12). The band amplified by PCR was confirmed by 1.0% agarose gel electrophoresis and ethidium bromide staining.

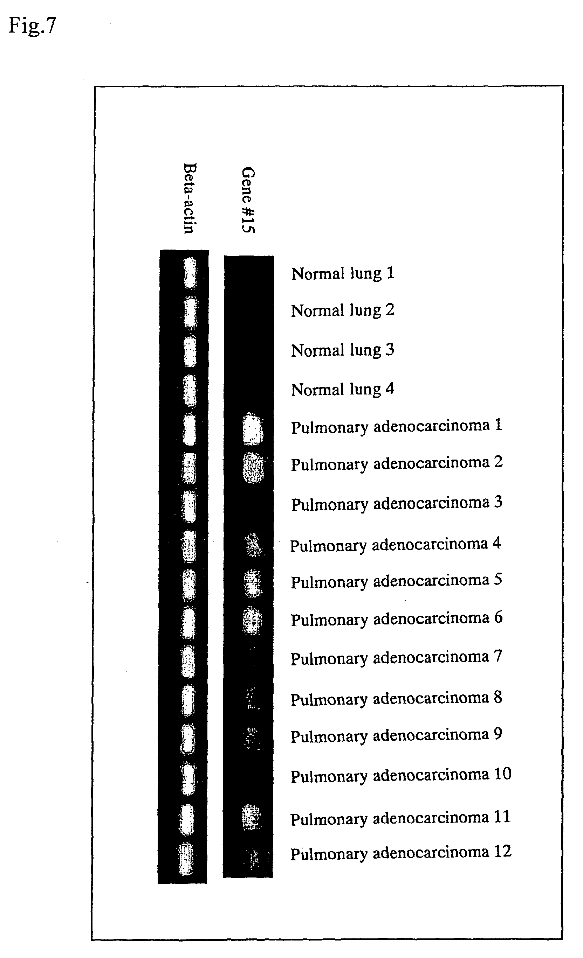

As a result, as shown in Figure 7, no amplification by PCR was observed in the 4 normal lung cases, while in pulmonary adenocarcinoma tissue amplification of a specific band was confirmed in 10 out of the 12 cases analyzed. These results confirm that expression of gene #15 mRNA is elevated with high frequency in pulmonary adenocarcinoma tissue.

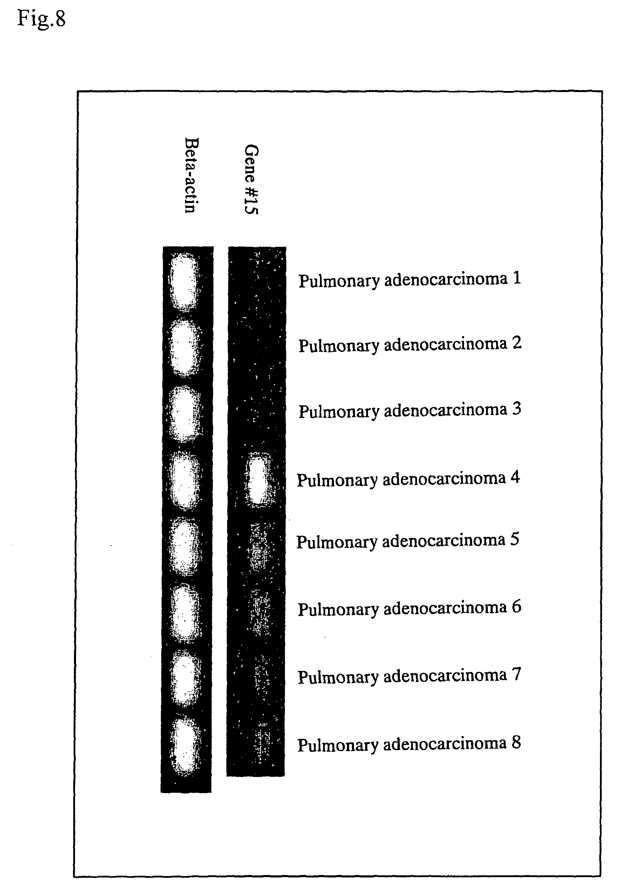

On the other hand it is known that lung cancer and other cancer tissues are contaminated by infiltrating lymphocytes, connective tissue and other tissue in addition to pulmonary adenocarcinoma cells, so it is necessary to clarify whether expression of gene #15 is elevated in all cells. Therefore, cancer cells alone were first isolated from pulmonary adenocarcinoma tissue by microdissection, and expression of gene #15 mRNA was confirmed as above by RT-PCR. That is, using an LM200 (LCM, Olympus) according to the attached directions lung cancer cells were microdissected from pulmonary adenocarcinoma tissue, total RNA was prepared from the isolated cancer cells, and single-strand cDNA was then synthesized. Next, amplification by PCR was attempted as described above. As a result, as shown in Figure 8, a specific amplification band for gene #15 was detected in the microdissected lung cancer cells.

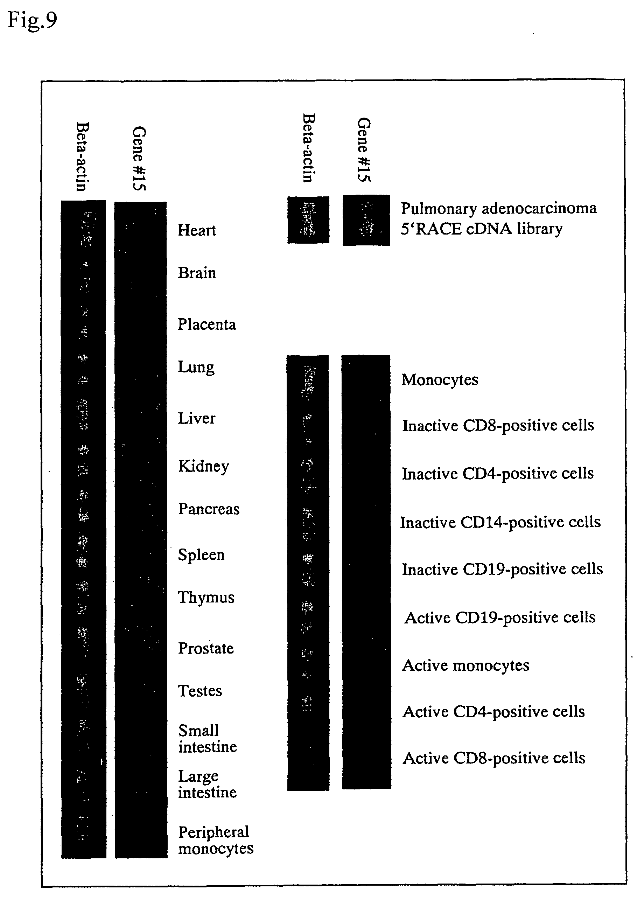

Next, in order to discover whether expression of gene #15 is elevated in lymphocytes which have infiltrated lung cancer tissue, Multiple Tissue cDNA Panel Human Blood Fractions (Clontech) comprising cDNA prepared from various inactive and active immune cells were used as template DNA for PCR performed as above. About 2/3 of the infiltrating immune cells in lung cancer tissue are lymphocytes, of which 80% are T lymphocytes with the remainder being B lymphocytes. The remaining 1/3 are thought to be infiltrating macrophages, with only a few NK cells and dendritic cells being reported (Agapi Kataki et al, *J Lab Clin Med* vol 140, 320-328, 2002). As a result, as shown in Figure 9, no expression of gene #15 was seen in either the active or inactive monocytes or lymphocytes. These results suggest the possibility that expression of gene #15 is specifically elevated in the pulmonary adenocarcinoma cells of pulmonary adenocarcinoma tissue.

### (2) Gene #15 expression analysis in normal human tissue

Expression of gene #15 in normal human tissue was analyzed by quantitative PCR as above. In this case, Multiple Tissue cDNA Panels Human I, II (Clontech) were used as the single-stranded cDNA prepared from normal human tissues. The single-stranded cDNA prepared above for 5' RACE was used as the positive control.

As a result, as shown in Figure 9, no expression of gene #15 was seen in any of the normal human tissues. These results match the results of the aforementioned Gene chip analysis.

### (3) mRNA expression analysis of gene #15 in human stomach cancer, hepatoma and large intestinal cancer.

Total RNA was prepared as above from progressive and differentiated stomach cancer (intestinal) (3 cases), moderately differentiated hepatoma derived from hepatitis C (3 cases), slightly differentiated hepatoma derived from hepatitis C (3 cases) and progressive large intestinal cancer (3 cases), equal amounts were mixed, and gene #15 expression analysis was performed as above using a GeneChip™ HG-U133B (Affymetryx).

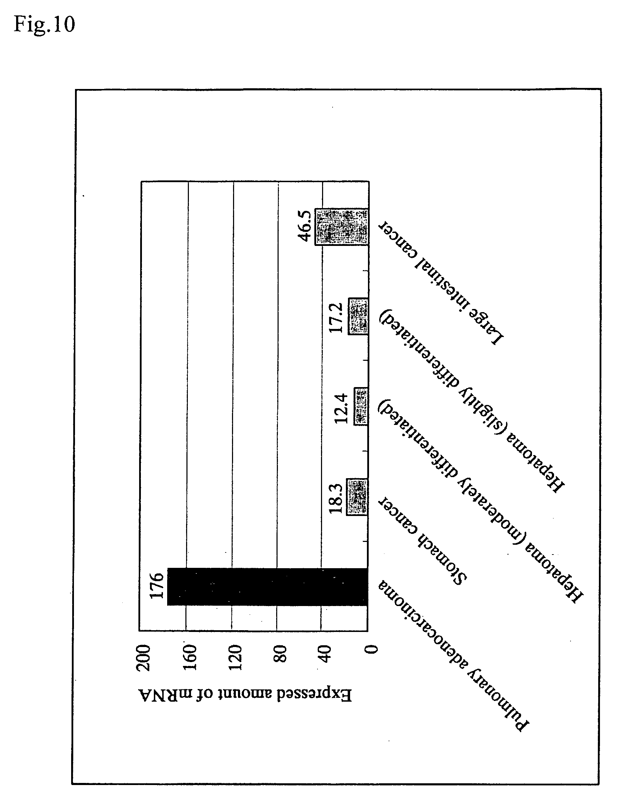

As a result, as shown in Figure 10, expression of mRNA reacting with the 230349_at_u133B probe was not seen in either the lung cancer, hepatoma or large intestinal cancer. It is therefore clear that expression of gene #15 is specifically elevated in pulmonary adenocarcinoma.

From these results it is clear that expression of the gene #15 which was identified here is specifically elevated with high frequency in pulmonary adenocarcinoma tissue, suggesting that gene #15 is a useful gene for diagnosing lung cancer and pulmonary adenocarcinoma in particular when used in gene expression analysis such as Gene chip analysis and RT-PCR. Moreover, because the gene expression pattern of primary lung cancer tissue tends to differ from that of metastatic lung cancer tissue occurring due to metastasis from large intestinal cancer, stomach cancer and the like (Bhattacharjee, A. et al, *Proc. Natl. Acad. Sci. USA* 98, p. 13790-13795, 2001), and since it appears that expression of this gene #15 is not elevated in metastatic lung cancers derived from large intestinal cancer and stomach cancer, it might be possible to diagnose primary lung cancer using this gene as the marker.

### INDUSTRIAL APPLICABILITY

A novel protein expression of which is specifically elevated in abnormal cells and abnormal tissue (particularly lung cancer cells and lung cancer tissue), a gene encoding that protein, a recombinant vector comprising that gene, a transformant comprising that recombinant vector and an antibody to the aforementioned protein are provided by the present invention. Moreover, a diagnostic method and diagnostic kit for diagnosing diseases involving elevated expression of a gene encoding a protein the expression of which is specifically elevated in abnormal cells and abnormal tissue (particularly lung cancer cells and lung cancer tissue) using as the marker the level of expression of that gene are provided by the present invention. Moreover, a screening method and screening kit for substances for preventing and treating diseases involving elevated expression of a gene encoding a protein the expression of which is specifically elevated in abnormal cells and abnormal tissue (particularly lung cancer cells and lung cancer tissue) using as the marker the reduction effect on the level of expression of that gene are provided by the present invention.

## Claims

1. A protein shown in (a) or (b) below.
(a) A protein comprising the amino acid sequence represented by Seq. ID No. 2
(b) A protein comprising the amino acid sequence represented by Seq. ID No. 2 with 1 or more amino acids deleted, replaced or added, the expression of which is specifically elevated in abnormal cells or abnormal tissue.

2. The protein according to Claim 1 wherein the abnormal cells or abnormal tissue are lung cancer cells or lung cancer tissue.

3. A gene encoding a protein according to Claim 1 or 2.

4. The gene according to Claim 3 comprising DNA shown in (c) or (d) below.
(c) DNA comprising the sequence of nucleotides 103 through 1488 in the nucleotide sequence represented by Seq. ID NO.1
(d) DNA which hybridizes under stringent conditions with DNA complementary to the DNA shown in (c) above, and which encodes a protein the expression of which is specifically elevated in abnormal cells or abnormal tissue.

5. A recombinant vector comprising the gene according to Claim 3 or 4.

6. A transformant comprising the recombinant vector according to Claim 5.

7. An antibody or fragment thereof capable of reacting to the protein according to Claim 1 or 2.

8. A diagnostic method for a disease involving elevated expression of a gene encoding the protein according to Claim 1, comprising a step of using as the indicator the level of expression of the gene in a specimen collected from a test animal to determine whether or not the test animal suffers from the disease.

9. The diagnostic method according to Claim 8, comprising a step of measuring the level of expression based on the amount of mRNA encoding the protein according to Claim 1 which is present in the specimen.

10. The diagnostic method according to Claim 8, comprising a step of measuring the level of expression based on the amount of the protein according to Claim 1 which is present in the specimen.

11. The diagnostic method according to any of Claims 8 through 10, wherein the disease is lung cancer.

12. A diagnostic kit for a disease involving elevated expression of a gene encoding the protein according to Claim 1, comprising an oligonucleotide or polynucleotide capable of hybridizing with a nucleic acid encoding the protein according to Claim 1.

13. A diagnostic kit for a disease involving elevated expression of a gene encoding the protein according to Claim 1, comprising an antibody or fragment thereof capable of reacting to the protein according to Claim 1.

14. The diagnostic kit according to Claim 12 or 13, wherein the disease is lung cancer.

15. A screening method for substances for preventing or treating a disease involving elevated expression of a gene encoding the protein according to Claim 1, comprising a step of evaluating the preventative and therapeutic effects of candidate substances on the disease using as the indicator the reduction effect on the level of expression of the gene in cells or tissue in which the gene is highly expressed.

16. The screening method according to Claim 15, wherein the disease is lung cancer.

17. A screening kit for substances for preventing or treating a disease involving elevated expression of a gene encoding the protein according to Claim 1, comprising an oligonucleotide or polynucleotide capable of hybridizing with a nucleic acid encoding the protein.

18. A screening kit for substances for preventing or treating a disease involving elevated expression of a gene encoding the protein according to Claim 1, comprising an antibody or fragment capable of reacting to the protein.

19. The screening kit according to Claim 17 or 18, wherein the disease is lung cancer.


SEQUENCE LISTING

<110> TOUDAI TLO, Ltd.

<110> CHUGAI PHARMACEUTICAL CO., LTD.

<120> Novel protein and gene coding the same

<130> 03768371.1

<160> 13

<210> 1

<211> 2408

<212> DNA

<213> Homo sapiens

<220>

<221> CDS

<222> (103)..(1491)

<400> 1

tagtgaatct ctgttcccaa actggacttg acagagctct cctcacctat acttggactg 60

tagcggccat agggttctct tggggatggg tgggagggtg ct atg aac aca aga 114
Met Asn Thr Arg
1

cca caa cat tca gaa aga acc tcg aca atg gac aga gtt tat gaa att 162
Pro Gln His Ser Glu Arg Thr Ser Thr Met Asp Arg Val Tyr Glu Ile
5                   10                  15                  20

cct gag gag cca aat gtg gat ccg gtt tca tct ctg gag gaa gat gtc 210
Pro Glu Glu Pro Asn Val Asp Pro Val Ser Ser Leu Glu Glu Asp Val
                25                  30                  35

atc cgt gga gcc aac ccc cga ttt act ttt cca ttt agc atc ctt ttc 258
Ile Arg Gly Ala Asn Pro Arg Phe Thr Phe Pro Phe Ser Ile Leu Phe
            40                  45                  50

tcc acc ttt ttg tac tgt ggg gag gct gca tct gct ttg tac atg gtt 306
Ser Thr Phe Leu Tyr Cys Gly Glu Ala Ala Ser Ala Leu Tyr Met Val
        55                  60                  65

aga atc tat cga aag aat agt gaa act tac tgg atg aca tac acc ttt 354
Arg Ile Tyr Arg Lys Asn Ser Glu Thr Tyr Trp Met Thr Tyr Thr Phe
    70                  75                  80

tct ttc ttt atg ttt tca tcc att atg gtc cag ttg acc ctc att ttt 402
Ser Phe Phe Met Phe Ser Ser Ile Met Val Gln Leu Thr Leu Ile Phe
85                  90                  95                  100

gtc cac aga gat cta gcc aaa gat aaa ccg cta tca tta ttt atg cat 450
Val His Arg Asp Leu Ala Lys Asp Lys Pro Leu Ser Leu Phe Met His
                105                 110                 115

cta atc ctc ttg gga cct gtt atc aga tgt ttg gag gcc atg att aag 498
Leu Ile Leu Leu Gly Pro Val Ile Arg Cys Leu Glu Ala Met Ile Lys
            120                 125                 130

tac ctc aca ctg tgg aag aaa gag gag cag gag gag ccc tat gtc agc 546

Tyr Leu Thr Leu Trp Lys Lys Glu Glu Gln Glu Glu Pro Tyr Val Ser
        135                 140                 145

ctc acc cga aag aag atg cta ata gat ggc gag gag gtg ctg ata gaa 594
Leu Thr Arg Lys Lys Met Leu Ile Asp Gly Glu Glu Val Leu Ile Glu
    150                 155                 160

tgg gag gtg ggc cac tcc atc cgg acc ctg gct atg cac cgc aat gcc 642
Trp Glu Val Gly His Ser Ile Arg Thr Leu Ala Met His Arg Asn Ala
165                 170                 175                 180

tac aaa cgt atg tca cag atc caa gcc ttc ctg ggc tca gtg ccc cag 690
Tyr Lys Arg Met Ser Gln Ile Gln Ala Phe Leu Gly Ser Val Pro Gln
                185                 190                 195

ctg acc tat cag ctc tat gtg agc ctg atc tct gca gag gtt ccc ctg 738
Leu Thr Tyr Gln Leu Tyr Val Ser Leu Ile Ser Ala Glu Val Pro Leu
            200                 205                 210

ggt aga gtt gtg cta atg gta ttt tcc ctg gta tct gtc acc tat ggg 786
Gly Arg Val Val Leu Met Val Phe Ser Leu Val Ser Val Thr Tyr Gly
        215                 220                 225

gcc acc ctt tgc aat atg ttg gct atc cag atc aag tac gat gac tac 834
Ala Thr Leu Cys Asn Met Leu Ala Ile Gln Ile Lys Tyr Asp Asp Tyr
    230                 235                 240

aag att cgc ctt ggg cca cta gaa gtc ctc tgc atc acc atc tgg cgg 882
Lys Ile Arg Leu Gly Pro Leu Glu Val Leu Cys Ile Thr Ile Trp Arg

245                 250                 255                 260

aca ttg gag atc act tcc cgc ctc ctg att ctg gtg ctc ttc tca gcc    930
Thr Leu Glu Ile Thr Ser Arg Leu Leu Ile Leu Val Leu Phe Ser Ala
                265                 270                 275

act ttg aaa ttg aag gct gtg ccc ttc cta gtg ctc aac ttc ctg atc    978
Thr Leu Lys Leu Lys Ala Val Pro Phe Leu Val Leu Asn Phe Leu Ile
            280                 285                 290

atc ctc ttt gag ccc tgg att aag ttc tgg aga agt ggt gcc cag atg    1026
Ile Leu Phe Glu Pro Trp Ile Lys Phe Trp Arg Ser Gly Ala Gln Met
        295                 300                 305

ccc aat aac att gag aaa aac ttc agc cgg gtc ggc act ctg gtg gtc    1074
Pro Asn Asn Ile Glu Lys Asn Phe Ser Arg Val Gly Thr Leu Val Val
    310                 315                 320

ctg att tca gtc acc atc ctc tat gct ggc atc aac ttc tct tgc tgg    1122
Leu Ile Ser Val Thr Ile Leu Tyr Ala Gly Ile Asn Phe Ser Cys Trp
325                 330                 335                 340

tca gct ttg cag ttg agg ttg gca gac aga gat ctc gtc gac aaa ggg    1170
Ser Ala Leu Gln Leu Arg Leu Ala Asp Arg Asp Leu Val Asp Lys Gly
                345                 350                 355

cag aac tgg gga cat atg ggc ctg cac tat agt gtg agg ttg gta gag    1218
Gln Asn Trp Gly His Met Gly Leu His Tyr Ser Val Arg Leu Val Glu
            360                 365                 370

aat gtg atc atg gtc ttg gtt ttt aag ttc ttt gga gtg aaa gtg tta 1266
Asn Val Ile Met Val Leu Val Phe Lys Phe Phe Gly Val Lys Val Leu
        375                 380                 385

ctg aat tac tgt cat tcc ttg att gcc ttg cag ctc att att gct tat 1314
Leu Asn Tyr Cys His Ser Leu Ile Ala Leu Gln Leu Ile Ile Ala Tyr
    390                 395                 400

ctg att tcc att ggc ttc atg ctc ctt ttc ttc cag tac ttg cat cca 1362
Leu Ile Ser Ile Gly Phe Met Leu Leu Phe Phe Gln Tyr Leu His Pro
405                 410                 415                 420

ttg cgc tca ctc ttc acc cat aat gta gta gac tac ctc cat tgt gtc 1410
Leu Arg Ser Leu Phe Thr His Asn Val Val Asp Tyr Leu His Cys Val
                425                 430                 435

tgc tgt cac cag cac cct cgg acc agg gtt gag aac tca gag cca ccc 1458
Cys Cys His Gln His Pro Arg Thr Arg Val Glu Asn Ser Glu Pro Pro
            440                 445                 450

ttt gag act gaa gca agg caa agt gtt gtc tga ttctattttc tgggtatttt 1511
Phe Glu Thr Glu Ala Arg Gln Ser Val Val
        455                 460

aggaagagtt gggagttgcc aagagtaacc atgaaattga acgaaaggat gaggttcatg 1571

ggtgagatac ccatcagtac attttcttga cttttctgtt aagcctatca gaagaaagag 1631

caactcccaa ataggtttta ttttcttaag agttaccact atgtttggaa acagggggta 1691

tcgactatat agttgaaagg gtcagaaata ccattcacac ccttcttacc caagtcaatt 1751

ggaataactt gtcttcaaac actttaggct ctctaaagtg accttctagc tctgctcatt 1811

tgcttgatgc atttctgagc tttcctgggc tgagctgaag gcccagaatc ccgctagaat 1871

atatcctgac tgatcagagg atatgacagc ttaccagcta agagtacctc ccaggaaaca 1931

gtctgactaa tgtggaacct gcaactgtca gtgtggctgg ggtcttttta attccagtga 1991

gaagctctgg ctgagaagaa aatcaccact attaaaaaag ctgctcccca agcagattag 2051

ctctctgtta ggattttact agtggccatt cagcaaggac ctctctttac agtggcactt 2111

cataggcaca ctctaaggag aaagtgcaga gtagaattcc ttcagggcat aagccaaaat 2171

gactcttttt ctcagggacc tgcatgggcc tccagcttgt ctattggaat tgttaagtga 2231

agcctctcac ttagtgcctc attagcagag atttcctcca acccagcttt tctgtgctct 2291

tggtatttta ctacttgatg tggacctcag agaagctgaa ctgtaattga aaatgtttcc 2351

gatgtgtgga agaaatgaag actgctttgt gaaaaaaaaa aaaaaaaaaa aaaaaaa 2408

<210> 2

<211> 462

<212> PRT
<213> Homo sapiens

<400> 2
Met Asn Thr Arg Pro Gln His Ser Glu Arg Thr Ser Thr Met Asp Arg
1               5                   10                  15
Val Tyr Glu Ile Pro Glu Glu Pro Asn Val Asp Pro Val Ser Ser Leu
            20                  25                  30
Glu Glu Asp Val Ile Arg Gly Ala Asn Pro Arg Phe Thr Phe Pro Phe
        35                  40                  45
Ser Ile Leu Phe Ser Thr Phe Leu Tyr Cys Gly Glu Ala Ala Ser Ala
    50                  55                  60
Leu Tyr Met Val Arg Ile Tyr Arg Lys Asn Ser Glu Thr Tyr Trp Met
65                  70                  75                  80
Thr Tyr Thr Phe Ser Phe Phe Met Phe Ser Ser Ile Met Val Gln Leu
                85                  90                  95
Thr Leu Ile Phe Val His Arg Asp Leu Ala Lys Asp Lys Pro Leu Ser
            100                 105                 110
Leu Phe Met His Leu Ile Leu Leu Gly Pro Val Ile Arg Cys Leu Glu
        115                 120                 125
Ala Met Ile Lys Tyr Leu Thr Leu Trp Lys Lys Glu Glu Gln Glu Glu
    130                 135                 140
Pro Tyr Val Ser Leu Thr Arg Lys Lys Met Leu Ile Asp Gly Glu Glu
145                 150                 155                 160
Val Leu Ile Glu Trp Glu Val Gly His Ser Ile Arg Thr Leu Ala Met
                165                 170                 175
His Arg Asn Ala Tyr Lys Arg Met Ser Gln Ile Gln Ala Phe Leu Gly
            180                 185                 190
Ser Val Pro Gln Leu Thr Tyr Gln Leu Tyr Val Ser Leu Ile Ser Ala

195                 200                 205
Glu Val Pro Leu Gly Arg Val Val Leu Met Val Phe Ser Leu Val Ser
    210                 215                 220
Val Thr Tyr Gly Ala Thr Leu Cys Asn Met Leu Ala Ile Gln Ile Lys
225                 230                 235                 240
Tyr Asp Asp Tyr Lys Ile Arg Leu Gly Pro Leu Glu Val Leu Cys Ile
                245                 250                 255
Thr Ile Trp Arg Thr Leu Glu Ile Thr Ser Arg Leu Leu Ile Leu Val
            260                 265                 270
Leu Phe Ser Ala Thr Leu Lys Leu Lys Ala Val Pro Phe Leu Val Leu
        275                 280                 285
Asn Phe Leu Ile Ile Leu Phe Glu Pro Trp Ile Lys Phe Trp Arg Ser
    290                 295                 300
Gly Ala Gln Met Pro Asn Asn Ile Glu Lys Asn Phe Ser Arg Val Gly
305                 310                 315                 320
Thr Leu Val Val Leu Ile Ser Val Thr Ile Leu Tyr Ala Gly Ile Asn
                325                 330                 335
Phe Ser Cys Trp Ser Ala Leu Gln Leu Arg Leu Ala Asp Arg Asp Leu
            340                 345                 350
Val Asp Lys Gly Gln Asn Trp Gly His Met Gly Leu His Tyr Ser Val
        355                 360                 365
Arg Leu Val Glu Asn Val Ile Met Val Leu Val Phe Lys Phe Phe Gly
    370                 375                 380
Val Lys Val Leu Leu Asn Tyr Cys His Ser Leu Ile Ala Leu Gln Leu
385                 390                 395                 400
Ile Ile Ala Tyr Leu Ile Ser Ile Gly Phe Met Leu Leu Phe Phe Gln
                405                 410                 415
Tyr Leu His Pro Leu Arg Ser Leu Phe Thr His Asn Val Val Asp Tyr
            420                 425                 430

Leu His Cys Val Cys Cys His Gln His Pro Arg Thr Arg Val Glu Asn
        435                 440                 445
Ser Glu Pro Pro Phe Glu Thr Glu Ala Arg Gln Ser Val Val
    450                 455                 460

<210> 3
<211> 24
<212> DNA
<213> Artificial Sequence

<220>
<223> sequence of PCR primer

<400> 3
ctcagccaga gcttctcact ggaa 24

<210> 4
<211> 24
<212> DNA
<213> Artificial Sequence

<220>
<223> sequence of PCR primer

<400> 4
cacactgaca gttgcaggtt ccac 24

<210> 5

<211> 25

<212> DNA

<213> Artificial Sequence

<220>

<223> sequence of PCR primer

<400> 5

atgtggaacc tgcaactgtc agtgt 25

<210> 6

<211> 25

<212> DNA

<213> Artificial Sequence

<220>

<223> sequece of PCR primer

<400> 6

gtgctatgaa cacaagacca caaca 25

<210> 7

<211> 25

<212> DNA

<213> Artificial Sequence

<220>

<223> sequece of PCR primer

<400> 7

aaggccatgg agaagaagga ggaaa 25

<210> 8

<211> 29

<212> DNA

<213> Artificial Sequence

<220>

<223> sequece of PCR primer

<400> 8

cagaaaatag aatcagacaa cactttgcc 29

<210> 9

<211> 25

<212> DNA

<213> Artificial Sequence

<220>

<223> sequece of PCR primer

<400> 9

tagagaatgt gatcatggtc ttggt 25

<210> 10
<211> 25
<212> DNA
<213> Artificial Sequence

<220>
<223> sequece of PCR primer

<400> 10
ggaggtagtc tactacatta tgggt 25

<210> 11
<211> 25
<212> DNA
<213> Artificial Sequence

<220>
<223> sequece of PCR primer

<400> 11
agaaggagat cactgccctg gcacc 25

<210> 12
<211> 25

<212> DNA

<213> Artificial Sequence

<220>

<223> sequece of PCR primer

<400> 12

cctgcttgct gatccacatc tgctg 25

<210> 13

<211> 495

<212> DNA

<213> Homo sapiens

<220>

<221> 5'UTR

<222> (1)..(472)

<400> 13

cttcttctgc gcccgctctt ctgccctggc tcagctctcc gctgacttga gaggacacac 60

tggtcaggac tctttgtgag gagctgctga gtgtcggtgc ccccgacaga tcggctacac 120

cctgcctgag gggctgcgaa aggagccgcc acggaagccg ctgttctcat gactcttcac 180

gtccctggag ttggactctg gatggggcgc tgggatgctt gcttttgtct tgttcaagtt 240

tcacagcaag tatgttgacg attggaatcg gggccaatca agagtcaagt tcaaagtggt 300

actcctgggc tttccatccc agactccaag tcgaatctga gtctagaaga gagcggtttc 360

ttgctctaac tagtgaatct ctgttcccaa actggacttg acagagctct cctcacctat 420

acttggactg tagcggccat agggttctct tggggatggg tgggagggtg ctatgaacac 480

aagaccacaa cattc 495